# EUROPEAN PATENT APPLICATION

(11) **EP 1 298 221 A1**
(43) Date of publication of application: **02.04.2003**
(21) Application number: 01203703.2
(22) Date of filing: 28.09.2001
(51) Int. Cl.: C12Q 1/68

(54) **Means and methods for treatment evaluation**

(71) Applicant: PrimaGen Holding B.V., 1105 BA Amsterdam (NL)
(72) Inventor: van der Kuyl, Antoinette C., 1231 VS Loosdrecht (NL); Cornelissen, Marion, 3642 AC Mijdrecht (NL)
(74) Representative: Prins, Adrianus Willem

(57) **Abstract**

The invention provides a method for determining whether a treatment is effective in changing the status of a certain set of target cells, such as a tumor, in a patient. This method implies obtaining a sample from a patient after initiation of a treatment, and determining whether said sample comprises an expression product of at least one marker gene. Preferably, said sample is a blood sample. In one aspect, said expression product is expressed by a peripheral blood mononuclear cell. Said marker gene may be a gene involved in the generation, maintenance and/or breakdown of blood vessels (angiogenesis). A method of the invention is very suitable to determine within a few days if a certain treatment against Kaposi's Sarcoma is successful. Moreover, this method is suitable for determining the presence of angiogenesis and/or tumor cells in a patient.

SEQUENCE LISTING

<110> PrimaGen B.V.

<120> Means and methods for treatment evaluation

<130> P57117EP00

<140> EP 01203703.2
<141> 2001-09-28

<160> 158

<170> PatentIn version 3.1

<210> 1
<211> 15
<212> DNA
<213> Artificial Sequence

<220>
<223> Tag with increased expression in SAGE libraries KS3 and KS4
<400> 1
catgccccag tcggc 15

<210> 2
<211> 15
<212> DNA
<213> Artificial Sequence

<220>
<223> Tag with increased expression in SAGE libraries KS3 and KS4
<400> 2
catgcttgac atacc 15

<210> 3

<211>  15

<212>  DNA

<213>  Artificial Sequence

<220>

<223>  Tag with increased expression in SAGE libraries KS3 and KS4

<400>  3
catgcatcac ggatc                                                        15

<210>  4

<211>  15

<212>  DNA

<213>  Artificial Sequence

<220>

<223>  Tag with increased expression in SAGE libraries KS3 and KS4

<400>  4
catgggccaa aggcc                                                        15

<210>  5

<211>  15

<212>  DNA

<213>  Artificial Sequence

<220>

<223>  Tag with increased expression in SAGE libraries KS3 and KS4

<400>  5
catgttgcat atcag                                                        15

<210>  6

<211>  15

<212>  DNA

<213>  Artificial Sequence

<220>

<223>  Tag with increased expression in SAGE libraries KS3 and KS4

<400>  6
catgccctgt tcagc                                                        15

<210> 7

<211> 15

<212> DNA

<213> Artificial Sequence

<220>

<223> Tag with increased expression in SAGE libraries KS3 and KS4

<400> 7
catggatcaa tcagt 15

<210> 8

<211> 15

<212> DNA

<213> Artificial Sequence

<220>

<223> Tag with increased expression in SAGE libraries KS3 and KS4

<400> 8
catggagggt gccaa 15

<210> 9

<211> 15

<212> DNA

<213> Artificial Sequence

<220>

<223> Tag with increased expression in SAGE libraries KS3 and KS4

<400> 9
catgtaaacc tgctg 15

<210> 10

<211> 15

<212> DNA

<213> Artificial Sequence

<220>

<223>  Tag with increased expression in SAGE libraries KS3 and KS4

<400>  10
catggtggcc agagg                                                        15

<210>  11

<211>  15

<212>  DNA

<213>  Artificial Sequence

<220>

<223>  Tag with increased expression in SAGE libraries KS3 and KS4

<400>  11
catgtctggc ccagc                                                        15

<210>  12

<211>  15

<212>  DNA

<213>  Artificial Sequence

<220>

<223>  Tag with increased expression in SAGE libraries KS3 and KS4

<400>  12
catgcaggtc gctac                                                        15

<210>  13

<211>  15

<212>  DNA

<213>  Artificial Sequence

<220>

<223>  Tag with increased expression in SAGE libraries KS3 and KS4

<400>  13
catggagcag cgccc                                                        15

<210>  14

<211>  15

<212>  DNA

<213>  Artificial Sequence

<220>

<223>  Tag with increased expression in SAGE libraries KS3 and KS4

<400>  14
catgacttat tatgc                                                        15

<210>  15

<211>  15

<212>  DNA

<213>  Artificial Sequence


<220>

<223>  Tag with increased expression in SAGE libraries KS3 and KS4

<400>  15
catgcaggcc tggcc                                                        15

<210>  16

<211>  15

<212>  DNA

<213>  Artificial Sequence


<220>

<223>  Tag with increased expression in SAGE libraries KS3 and KS4

<400>  16
catggtgcgg aggac                                                        15

<210>  17

<211>  15

<212>  DNA

<213>  Artificial Sequence


<220>

<223>  Tag with increased expression in SAGE libraries KS3 and KS4

<400>  17
catgacagcg gcaat                                                        15

<210>  18

<211>  15

<212>  DNA
<213>  Artificial Sequence

<220>
<223>  Tag with increased expression in SAGE libraries KS3 and KS4

<400>  18
catggatgtg cacga                                                        15

<210>  19
<211>  15
<212>  DNA
<213>  Artificial Sequence

<220>
<223>  Tag with increased expression in SAGE libraries KS3 and KS4

<400>  19
catgcaggtt tcata                                                        15

<210>  20
<211>  15
<212>  DNA
<213>  Artificial Sequence

<220>
<223>  Tag with increased expression in SAGE libraries KS3 and KS4

<400>  20
catgaactct gaccc                                                        15

<210>  21
<211>  15
<212>  DNA
<213>  Artificial Sequence

<220>
<223>  Tag with increased expression in SAGE libraries KS3 and KS4

<400>  21
catgaaatca ataca                                                        15

<210> 22

<211> 15

<212> DNA

<213> Artificial Sequence


<220>

<223> Tag with increased expression in SAGE libraries KS3 and KS4

<400> 22
catgtggtaa ctggc 15

<210> 23

<211> 15

<212> DNA

<213> Artificial Sequence


<220>

<223> Tag with increased expression in SAGE libraries KS3 and KS4

<400> 23
catgtctgca ctgag 15

<210> 24

<211> 15

<212> DNA

<213> Artificial Sequence


<220>

<223> Tag with increased expression in SAGE libraries KS3 and KS4

<400> 24
catgcaggct gctgg 15

<210> 25

<211> 15

<212> DNA

<213> Artificial Sequence


<220>

<223> Tag with increased expression in SAGE libraries KS3 and KS4

<400>  25
catgatgaca gatgg                                                        15

<210>  26
<211>  15
<212>  DNA
<213>  Artificial Sequence

<220>
<223>  Tag with increased expression in SAGE libraries KS3 and KS4

<400>  26
catggcacaa caaga                                                        15

<210>  27
<211>  15
<212>  DNA
<213>  Artificial Sequence

<220>
<223>  Tag with increased expression in SAGE libraries KS3 and KS4

<400>  27
catgccacag gagaa                                                        15

<210>  28
<211>  15
<212>  DNA
<213>  Artificial Sequence

<220>
<223>  Tag with increased expression in SAGE libraries KS3 and KS4

<400>  28
catgctgtgc ggaac                                                        15

<210>  29
<211>  15
<212>  DNA
<213>  Artificial Sequence

<220>

<223> Tag with increased expression in SAGE libraries KS3 and KS4

<400> 29
catggatggc tgcct 15

<210> 30

<211> 15

<212> DNA

<213> Artificial Sequence

<220>

<223> Tag with increased expression in SAGE libraries KS3 and KS4

<400> 30
catgctccat tgcca 15

<210> 31

<211> 15

<212> DNA

<213> Artificial Sequence

<220>

<223> Tag with increased expression in SAGE libraries KS3 and KS4

<400> 31
catgacctcc actgg 15

<210> 32

<211> 15

<212> DNA

<213> Artificial Sequence

<220>

<223> Tag with increased expression in SAGE libraries KS3 and KS4

<400> 32
catgcaggcc tggcc 15

<210> 33

<211> 46

<212> DNA

<213>  Artificial Sequence

<220>
<223>  oligo (dT) primer with a 5' M13 tail

<400>  33
ctagttgtaa aacgacggcc agtttttttt tttttttttt tttttt                    46

<210>  34
<211>  17
<212>  DNA
<213>  Artificial Sequence

<220>
<223>  primer

<400>  34
gtaaaacgac ggccagt                                                    17

<210>  35
<211>  20
<212>  DNA
<213>  Artificial Sequence

<220>
<223>  primer
<220>
<221>  misc_feature
<222>  (1)..(6)
<223>  "n" on positions 1-6 stands for "I" = inosine

<400>  35
nnnnncatga cctccactgg                                                 20

<210>  36
<211>  20
<212>  DNA
<213>  Artificial Sequence

<220>

<223>  primer used for tag confirmation

<220>

<221>  misc_feature

<222>  (1)..(6)

<223>  "n" on positions 1 - 6 stands for "I" = inosine


<400>  36
nnnnncatgg atgtgcacga                                                    20

<210>  37

<211>  20

<212>  DNA

<213>  Artificial Sequence


<220>

<223>  primer used for tag confirmation

<220>

<221>  misc_feature

<222>  (1)..(6)

<223>  "n" on positions 1 - 6 stands for "I" = inosine


<400>  37
nnnnncatgc cccagtcggc                                                    20

<210>  38

<211>  20

<212>  DNA

<213>  Artificial Sequence


<220>

<223>  primer used for tag confirmation

<220>

<221>  misc_feature

<222>  (1)..(6)

<223>  "n" on positions 1 - 6 stands for "I" = inosine

<400> 38
nnnnncatgc ttgacatacc 20

<210> 39
<211> 20
<212> DNA
<213> Artificial Sequence

<220>
<223> primer used for tag confirmation
<220>
<221> misc_feature
<222> (1)..(6)
<223> "n" on positions 1 - 6 stands for "I" = inosine

<400> 39
nnnnncatgc atcacggatc 20

<210> 40
<211> 20
<212> DNA
<213> Artificial Sequence

<220>
<223> primer used for tag confirmation
<220>
<221> misc_feature
<222> (1)..(6)
<223> "n" on positions 1 - 6 stands for "I" = inosine

<400> 40
nnnnncatgg gccaaaggcc 20

<210> 41
<211> 20
<212> DNA

<213>  Artificial Sequence

<220>
<223>  primer used for tag confirmation
<220>
<221>  misc_feature
<222>  (1)..(6)
<223>  "n" on positions 1 - 6 stands for "I" = inosine

<400>  41
nnnnncatgt tgcatatcag                                                    20

<210>  42
<211>  20
<212>  DNA
<213>  Artificial Sequence

<220>
<223>  primer used for tag confirmation
<220>
<221>  misc_feature
<222>  (1)..(6)
<223>  "n" on positions 1 - 6 stands for "I" = inosine

<400>  42
nnnnncatgc cctgttcagc                                                    20

<210>  43
<211>  20
<212>  DNA
<213>  Artificial Sequence

<220>
<223>  primer used for tag confirmation
<220>
<221>  misc_feature

<222>  (1)..(6)
<223>  "n" on positions 1 - 6 stands for "I" = inosine

<400>  43
   nnnnncatgg atcaatcagt                                                    20

<210>  44
<211>  20
<212>  DNA
<213>  Artificial Sequence

<220>
<223>  primer used for tag confirmation
<220>
<221>  misc_feature
<222>  (1)..(6)
<223>  "n" on positions 1 - 6 stands for "I" = inosine

<400>  44
   nnnnncatgg agggtgccaa                                                    20

<210>  45
<211>  20
<212>  DNA
<213>  Artificial Sequence

<220>
<223>  primer used for tag confirmation
<220>
<221>  misc_feature
<222>  (1)..(6)
<223>  "n" on positions 1 - 6 stands for "I" = inosine

<400>  45
   nnnnncatgt aaacctgctg                                                    20

<210>  46

<211>  20

<212>  DNA

<213>  Artificial Sequence


<220>

<223>  primer used for tag confirmation

<220>

<221>  misc_feature

<222>  (1)..(6)

<223>  "n" on positions 1 - 6 stands for "I" = inosine


<400>  46
nnnnncatgg tggccagagg                                                    20

<210>  47

<211>  20

<212>  DNA

<213>  Artificial Sequence


<220>

<223>  primer used for tag confirmation

<220>

<221>  misc_feature

<222>  (1)..(6)

<223>  "n" on positions 1 - 6 stands for "I" = inosine


<400>  47
nnnnncatgt ctggcccagc                                                    20

<210>  48

<211>  20

<212>  DNA

<213>  Artificial Sequence


<220>

<223>  primer used for tag confirmation

<220>

<221>  misc_feature
<222>  (1)..(6)
<223>  "n" on positions 1 - 6 stands for "I" = inosine


<400>  48
nnnnncatgc aggtcgctac                                                   20

<210>  49
<211>  20
<212>  DNA
<213>  Artificial Sequence


<220>
<223>  primer used for tag confirmation
<220>
<221>  misc_feature
<222>  (1)..(6)
<223>  "n" on positions 1 - 6 stands for "I" = inosine


<400>  49
nnnnncatgg agtagcgccc                                                   20

<210>  50
<211>  20
<212>  DNA
<213>  Artificial Sequence


<220>
<223>  primer used for tag confirmation
<220>
<221>  misc_feature
<222>  (1)..(6)
<223>  "n" on positions 1 - 6 stands for "I" = inosine


<400>  50

nnnnncatga cttattatgc                                                    20

<210>  51
<211>  20
<212>  DNA
<213>  Artificial Sequence

<220>
<223>  primer used for tag confirmation
<220>
<221>  misc_feature
<222>  (1)..(6)
<223>  "n" on positions 1 - 6 stands for "I" = inosine

<400>  51
nnnnncatgc aggcctggcc                                                    20

<210>  52
<211>  20
<212>  DNA
<213>  Artificial Sequence

<220>
<223>  primer used for tag confirmation
<220>
<221>  misc_feature
<222>  (1)..(6)
<223>  "n" on positions 1 - 6 stands for "I" = inosine

<400>  52
nnnnncatgg tgcggaggac                                                    20

<210>  53
<211>  20
<212>  DNA
<213>  Artificial Sequence

<220>

<223> primer used for tag confirmation

<220>

<221> misc_feature

<222> (1)..(6)

<223> "n" on positions 1 - 6 stands for "I" = inosine


<400> 53
nnnnncatga cagcggcaat 20

<210> 54

<211> 20

<212> DNA

<213> Artificial Sequence


<220>

<223> primer used for tag confirmation

<220>

<221> misc_feature

<222> (1)..(6)

<223> "n" on positions 1 - 6 stands for "I" = inosine


<400> 54
nnnnncatgc aggtttcata 20

<210> 55

<211> 20

<212> DNA

<213> Artificial Sequence


<220>

<223> primer used for tag confirmation

<220>

<221> misc_feature

<222> (1)..(6)

<223> "n" on positions 1 - 6 stands for "I" = inosine

<400>  55
nnnnncatga actctgaccc                                                    20

<210>  56
<211>  20
<212>  DNA
<213>  Artificial Sequence

<220>
<223>  primer used for tag confirmation
<220>
<221>  misc_feature
<222>  (1)..(6)
<223>  "n" on positions 1 - 6 stands for "I" = inosine

<400>  56
nnnnncatga aatcaataca                                                    20

<210>  57
<211>  20
<212>  DNA
<213>  Artificial Sequence

<220>
<223>  primer used for tag confirmation
<220>
<221>  misc_feature
<222>  (1)..(6)
<223>  "n" on positions 1 - 6 stands for "I" = inosine

<400>  57
nnnnncatgt ggtaactggc                                                    20

<210>  58
<211>  20
<212>  DNA

<213>  Artificial Sequence

<220>
<223>  primer used for tag confirmation
<220>
<221>  misc_feature
<222>  (1)..(6)
<223>  "n" on positions 1 - 6 stands for "I" = inosine

<400>  58
nnnnncatgt ctgcactgag                                                    20

<210>  59
<211>  20
<212>  DNA
<213>  Artificial Sequence

<220>
<223>  primer used for tag confirmation
<220>
<221>  misc_feature
<222>  (1)..(6)
<223>  "n" on positions 1 - 6 stands for "I" = inosine

<400>  59
nnnnncatgc aggctgctgg                                                    20

<210>  60
<211>  20
<212>  DNA
<213>  Artificial Sequence

<220>
<223>  primer used for tag confirmation
<220>
<221>  misc_feature

<222>  (1)..(6)
<223>  "n" on positions 1 - 6 stands for "I" = inosine

<400>  60
nnnnncatga tgacagatgg                                                    20

<210>  61
<211>  20
<212>  DNA
<213>  Artificial Sequence

<220>
<223>  primer used for tag confirmation
<220>
<221>  misc_feature
<222>  (1)..(6)
<223>  "n" on positions 1 - 6 stands for "I" = inosine

<400>  61
nnnnncatgg cacaacaaga                                                    20

<210>  62
<211>  20
<212>  DNA
<213>  Artificial Sequence

<220>
<223>  primer used for tag confirmation
<220>
<221>  misc_feature
<222>  (1)..(6)
<223>  "n" on positions 1 - 6 stands for "I" = inosine

<400>  62
nnnnncatgc cacaggagaa                                                    20

<210>  63

<211>  20

<212>  DNA

<213>  Artificial Sequence

<220>

<223>  primer used for tag confirmation

<220>

<221>  misc_feature

<222>  (1)..(6)

<223>  "n" on positions 1 - 6 stands for "I" = inosine

<400>  63
nnnnncatgc tgtgcggaac                                                    20

<210>  64

<211>  20

<212>  DNA

<213>  Artificial Sequence

<220>

<223>  primer used for tag confirmation

<220>

<221>  misc_feature

<222>  (1)..(6)

<223>  "n" on positions 1 - 6 stands for "I" = inosine

<400>  64
nnnnncatgg atggctgcct                                                    20

<210>  65

<211>  20

<212>  DNA

<213>  Artificial Sequence

<220>

<223>  primer used for tag confirmation

<220>

<221> misc_feature

<222> (1)..(6)

<223> "n" on positions 1 - 6 stands for "I" = inosine


<400> 65
nnnnncatgc tccattgcca                                                    20

<210> 66

<211> 20

<212> DNA

<213> Artificial Sequence


<220>

<223> primer used for tag confirmation

<220>

<221> misc_feature

<222> (1)..(6)

<223> "n" on positions 1 - 6 stands for "I" = inosine


<400> 66
nnnnncatga cctccactgg                                                    20

<210> 67

<211> 100

<212> DNA

<213> Artificial Sequence


<220>

<223> TAG004

<400> 67
catgacctcc actggaagag ggggctagcg tgagcgctga ttctcaacct accataactc      60

tttcctgcct caggaactcc aataaaacat tttccatcca                            100

<210> 68

<211> 242

<212> DNA

<213>  Artificial Sequence

<220>
<223>  TAG007
<400>  68
catggatgtg cacgatggca aggtggtgtc cacccacgag caggtccttc gcaccaagaa      60
ctgaggctgc ccagccccgc tcaggcctag gaggcccccc gtgtggacac agatcccact     120
ggaagatccc ctctcctgcc caagcacttc acagctggac cctgcttcac cctcaccccc     180
tcctggcaat caatacagct tcattatctg agttgctaaa aaaaaaaaaa aaaaaaaaaa     240
aa                                                                    242

<210>  69
<211>  240
<212>  DNA
<213>  Artificial Sequence

<220>
<223>  TAG010
<400>  69
atctaccagc tcatgatgca gtgctggcag caggagcgtg cccaccgccc caagttcgct      60
gacatcgtca gcatcctgga caagctcatt cgtgcccctg actccctcaa gaccctggct     120
gactttgacc cccgcgtgtc tatccggctc cccagcacga gcggctcgga gggggtgccc     180
ttccgcacgg tgtccgagtg gctggagtcc atcaagatgc agcagtatac ggagcacttc     240

<210>  70
<211>  355
<212>  DNA
<213>  Artificial Sequence

<220>
<223>  TAG011
<400>  70
catgcttgac atacctacca gtattattcc cgacgacaca tatacatatg agaatatacc      60
ttatttattt ttgtgtaggt gtctgccttc acaaatgtca ttgtctactc ctagaagaac     120
caaatacctc aatttttgtt tttgagtact gtactatcct gtaaatatat cttaagcagg     180
tttgttttca gcactgatgg aaaataccag tgttgggttt ttttttagtt gccaacagtt     240

gtatgtttgc tgattattta tgacctgaaa taatatattt cttcttctaa gaagacattt     300

tgttacataa ggatgacttt tttatacaat ggaataaatt atggcatttc tattg          355

<210>  71

<211>  63

<212>  DNA

<213>  Artificial Sequence

<220>

<223>  TAG012

<400>  71
catgcatcac ggatcaatag actgtactta ttttccaata aaattttcaa actttgtact     60

gtt                                                                   63

<210>  72

<211>  212

<212>  DNA

<213>  Artificial Sequence

<220>

<223>  TAG013

<400>  72
aacttgccct gtgcctgtgt cccccatgct aggggcggag gggtcttttc cttcttcttt     60

cctacctacc ccttttctct tggccagggg cctcgtatcc tacctttcct tgtcccctgg    120

gctggctgca cagaggattg ccccttctct tttcagagct ggccctcgat gccaaattag    180

catttagtat tttgctcaaa gtctaaggga cc                                  212

<210>  73

<211>  214

<212>  DNA

<213>  Artificial Sequence

<220>

<223>  TAG014

<400>  73
   catgttgcat atcagggtgc tcaaggattg gagaggagac aaaaccagga gcagcacagt     60
   ggggacatct cccgtctcaa cagccccagg cctatggggg ctctggaagg atgggccagc    120
   ttgcaggggt tggggaggga gacatccagc ttgggctttc ccctttggaa taaaccattg 180
   gtctgtcaca aaaaaaaaaa aaaaaaaaaa aaaa 214

<210> 74

<211> 93

<212> DNA

<213> Artificial Sequence

<220>

<223> TAG015

<400> 74
   catgccctgt tcagctactc ccactcccgg cctgtcattc agaaaaaaat aaatgttcta 60
   ataagctcca aaaaaaaaaa aaaaaaaaaa aaa 93

<210> 75

<211> 163

<212> DNA

<213> Artificial Sequence

<220>

<223> TAG016

<400> 75
catggatcaa tcagtgtgat tagctttctc agcagacatt gtgccatatg tatcaaatga 60

caaatcttta ttgaatggtt ttgctcagca ccacctttta atatattggc agtacttatt 120

atataaaagg taaaccagca ttctcaaaaa aaaaaaaaaa aaa 163

<210> 76

<211> 205

<212> DNA

<213> Artificial Sequence

<220>

<223> TAG017

<400> 76
catggagggt gccaacagca tcttttccgg gttcctgctc tttccagata tggaggcctg 60

acctgtgggc tgcttcacat ccaccccggc tccccctgcc agcaacgctc actctacccc 120

caacaccacc ccttgcccag ccaatgcaca cagtagggct tggtgaatgc tgctgagtga 180

atgagtaaat aaactcttca aggcc 205

<210> 77

<211> 175

<212> DNA

<213> Artificial Sequence

<220>

<223> TAG018

<400> 77
cggctggaca cgtcggaggt ggtcttcaac agcaaggagc aaggctcctg gggccgcgag 60

gagcgcgggc cgggcgttcc tttccagcgc gggcagccct tcgaggtgct catcatcgcg 120

tcagacgacg gcttcaaggc cgtggttggg gacgcccagt accaccactt ccgcc 175

<210> 78

<211> 204

<212> DNA

<213> Artificial Sequence

<220>

<223> TAG019

<400> 78
cacaacctcg actactacaa gaagacaacc aacggccggc tgcccgtgaa gtggatggcg 60

cctgaggcat tatttgaccg agtctacact caccagagtg acgtctggtc ctttggggtc 120

ctgctctggg agatcttcac gctggggggc tccccgtacc ccggcatccc tgtggaggag 180

ctcttcaagc tgctgaagga gggc 204

<210> 79

<211> 95

<212> DNA

<213> Artificial Sequence

<220>

<223> TAG022

<400> 79
catggagcag cgccctgttc cgggggcagc cagtgaccca gccccaccaa tgggcctcca 60

gagaccccag gaacaataaa atgtcttctc ccacc 95

<210> 80

<211> 139

<212> DNA

<213> Artificial Sequence

<220>

<223> TAG025

<400> 80
catgtctgca ctgagaaact gcatttcagt agcatttgtc atccagccgg aagttaaagc 60

acacttactt tattcaccta tttttataat aaacgttctt gctgctgtga aaaaaaaaaa 120

aaaaaaaaaa aaaaaaaaa 139

<210> 81

<211> 234

<212> DNA

<213> Artificial Sequence

<220>

<223> TAG029

<400> 81
catgccacag gagaattcgg ggatttgagt ttctctgaat agcatatata tgatgcatcg 60

gataggtcat tatgattttt taccatttcg acttacataa tgaaaaccaa ttcattttaa 120

atatcagatt attattttgt aagttgtgga aaaagctaat tgtagttttc attatgaagt 180

tttcccaata aaccaggtat tctaaacttg aaaaaaaaaa aaaaaaaaaa aaaa 234

<210> 82

<211> 194

<212> DNA

<213> Artificial Sequence

<220>

<223> TAG030

<400> 82
catgctgtgc ggaactgcgt cagggcaaat gtcacagcag gatttcccca acccagctcc 60

atcatcacag acacagaggg ctgcagggga ggcctgccca ctgttttgtc gactctgccc 120

tcctctggca gcatagatcc ttaggtgctc aataaaggtg tgctgtattg aaaaaaaaaa 180

aaaaaaaaaa aaaa 194

<210> 83

<211> 97

<212> DNA

<213> Artificial Sequence

<220>

<223> TAG032

<400> 83
catgctccat tgccagactc ttgctgggag cccgtccaga atgtcctccc aataaaactc 60

catcctatga cgcaaaaaaa aaaaaaaaaa aaaaaaa 97

<210> 84

<211> 143

<212> DNA

<213> Artificial Sequence

<220>

<223> TAG036

<400> 84
catgacagcg gcaatctttt ctttggtcaa agttttctgt ttattttgct tgtcatattc 60

gatgtacttt aaggtgtctt tatgaagttt gctattctgg caataaactt ttagacttta 120

aaaaaaaaaa aaaaaaaaaa aaa 143

<210> 85

<211> 21

<212> DNA

<213> Artificial Sequence

<220>

<223> primer 5'TAG004GENE

<400> 85
ggcctttaac accccgttcc t 21

<210> 86

<211> 24

<212> DNA

<213>  Artificial Sequence

<220>

<223>  primer 3'TAG004GENE

<400>  86
tggtaggttg agaatcagcg ctca                                              24

<210>  87

<211>  24

<212>  DNA

<213>  Artificial Sequence

<220>

<223>  primer 5'TAG007GENE-N

<400>  87
aggagaccaa aggtcgctac tgca                                              24

<210>  88

<211>  22

<212>  DNA

<213>  Artificial Sequence

<220>

<223>  primer 3'TAG007GENE

<400>  88
cagttcttgg tgcgaaggac ct                                                22

<210>  89

<211>  25

<212>  DNA

<213>  Artificial Sequence

<220>

<223>  primer 5'TAG010GENE

<400>  89
atctaccagc tcatgatgca gtgct                                             25

<210>  90

<211>  24
<212>  DNA
<213>  Artificial Sequence

<220>
<223>  primer 3'TAG010GENE
<400>  90
gaagtgctcc gtatactgct gcat                                              24

<210>  91
<211>  24
<212>  DNA
<213>  Artificial Sequence

<220>
<223>  primer 5'TAG011GENE
<400>  91
agtgggtaca tcaagtccat ctga                                              24

<210>  92
<211>  23
<212>  DNA
<213>  Artificial Sequence

<220>
<223>  primer 3'TAG011GENE
<400>  92
cactggtatt ttccatcagt gct                                               23

<210>  93
<211>  24
<212>  DNA
<213>  Artificial Sequence

<220>
<223>  primer 5'TAG012GENE
<400>  93

taaagttgtc ctgcttgagc tgga 24

<210> 94
<211> 24
<212> DNA
<213> Artificial Sequence

<220>
<223> primer 3'TAG012GENE
<400> 94
ggcacgtgag cctctctttg cagt 24

<210> 95
<211> 24
<212> DNA
<213> Artificial Sequence

<220>
<223> primer 5'TAG013GENE
<400> 95
ctctacccca gaggaattta caga 24

<210> 96
<211> 24
<212> DNA
<213> Artificial Sequence

<220>
<223> primer 3'TAG013GENE
<400> 96
gggccagacc aaacacagac ctct 24

<210> 97
<211> 22
<212> DNA
<213> Artificial Sequence

<220>

<223>  primer 5'TAG014GENE

<400>  97
ggcaacaagc agaaggcggt ca                                                22

<210>  98

<211>  24

<212>  DNA

<213>  Artificial Sequence


<220>

<223>  primer 3'TAG014GENE

<400>  98
tgatcttgag ctgcagctgc tcct                                              24

<210>  99

<211>  21

<212>  DNA

<213>  Artificial Sequence


<220>

<223>  primer 5'TAG015GENE

<400>  99
gaatgtgctg gtcggagaga a                                                 21

<210>  100

<211>  22

<212>  DNA

<213>  Artificial Sequence


<220>

<223>  primer 3'TAG015GENE

<400>  100
tggggcagct tttcatagag ct                                                22

<210>  101

<211>  24

<212>  DNA

<213>  Artificial Sequence

<220>

<223>  primer 5'TAG016GENE

<400>  101
ttctctgcct gcccagcatc atga                                              24

<210>  102

<211>  24

<212>  DNA

<213>  Artificial Sequence


<220>

<223>  primer 3'TAG016GENE

<400>  102
tcaggcattc agcttcaggt cgct                                              24

<210>  103

<211>  22

<212>  DNA

<213>  Artificial Sequence


<220>

<223>  primer 5'TAG017GENE

<400>  103
gtctctacta cttcacctac ca                                                22

<210>  104

<211>  25

<212>  DNA

<213>  Artificial Sequence


<220>

<223>  primer 3'TAG017GENE

<400>  104
tgttgggggt agagtgagcg ttgct                                             25

<210>  105

<211>  24

<212>  DNA
<213>  Artificial Sequence

<220>
<223>  primer 5'TAG018GENE

<400>  105
gcaggttcca tgtaaacctg ctgt                                              24

<210>  106
<211>  24
<212>  DNA
<213>  Artificial Sequence

<220>
<223>  primer 3'TAG018GENE

<400>  106
ctgctcagaa gatcctcacg gagt                                              24

<210>  107
<211>  25
<212>  DNA
<213>  Artificial Sequence

<220>
<223>  primer 5'TAG019GENE

<400>  107
gtgaccgagg acaacgtgat gaaga                                             25

<210>  108
<211>  24
<212>  DNA
<213>  Artificial Sequence

<220>
<223>  primer 3'TAG019GENE

<400>  108
catgatcatg tacaggtcgt gtgt                                              24

<210> 109

<211> 22

<212> DNA

<213> Artificial Sequence

<220>

<223> primer 5'TAG022GENE

<400> 109
tgagcaacac tcaagctgag ag 22

<210> 110

<211> 19

<212> DNA

<213> Artificial Sequence

<220>

<223> primer 3'TAG022GENE

<400> 110
tctctggagg cccattggt 19

<210> 111

<211> 24

<212> DNA

<213> Artificial Sequence

<220>

<223> primer 5'TAG025GENE

<400> 111
atggggtcag gaacatctgg caga 24

<210> 112

<211> 24

<212> DNA

<213> Artificial Sequence

<220>

<223> primer 3'TAG025GENE

<400> 112
tccggctgga tgacaaatgc tact 24

<210> 113
<211> 24
<212> DNA
<213> Artificial Sequence

<220>
<223> primer 5'TAG029GENE

<400> 113
ctcaggttta tctgggctct atca 24

<210> 114
<211> 23
<212> DNA
<213> Artificial Sequence

<220>
<223> primer 3'TAG029GENE

<400> 114
tcataatgac ctatccgatg cat 23

<210> 115
<211> 24
<212> DNA
<213> Artificial Sequence

<220>
<223> primer 5'TAG030GENE

<400> 115
cttgcaaaga taggagaggc tcca 24

<210> 116
<211> 24
<212> DNA
<213> Artificial Sequence

<220>

<223>  primer 3'TAG030GENE

<400>  116
attgagcacc taaggatcta tgct                                              24

<210>  117

<211>  24

<212>  DNA

<213>  Artificial Sequence


<220>

<223>  primer 5'TAG032GENE

<400>  117
tgcgaatcag ggaccaacag gaga                                              24

<210>  118

<211>  24

<212>  DNA

<213>  Artificial Sequence


<220>

<223>  primer 3'TAG032GENE

<400>  118
ttgggaggac attctggacg ggct                                              24

<210>  119

<211>  24

<212>  DNA

<213>  Artificial Sequence


<220>

<223>  primer 5'TAG036GENE

<400>  119
atttagcagt agttctattg ggca                                              24

<210>  120

<211>  24

<212>  DNA

<213>  Artificial Sequence

<220>

<223>  primer 3'TAG036GENE

<400>  120
actgattagc acttcagacg cact                                              24

<210>  121

<211>  19

<212>  DNA

<213>  Artificial Sequence

<220>

<223>  primer 5'TAG004GENE-2

<400>  121
catcgacaaa ttgcgatct                                                    19

<210>  122

<211>  20

<212>  DNA

<213>  Artificial Sequence

<220>

<223>  primer 3'TAG004GENE-2

<400>  122
cgctagcccc ctcttccagt                                                   20

<210>  123

<211>  19

<212>  DNA

<213>  Artificial Sequence

<220>

<223>  primer 5'TAG007GENE-2.1

<400>  123
aggagatgat tggcagcgt                                                    19

<210>  124

<211>  22

<212>  DNA

<213>  Artificial Sequence

<220>

<223>  primer 3'TAG007GENE-2

<400>  124
ggaggaggtc acatctctgg at                                                22

<210>  125

<211>  19

<212>  DNA

<213>  Artificial Sequence

<220>

<223>  primer 5'TAG010GENE-2

<400>  125
ccaagttcgc tgacatcgt                                                    19

<210>  126

<211>  21

<212>  DNA

<213>  Artificial Sequence

<220>

<223>  primer 3'TAG010GENE-2

<400>  126
tgctggggag ccggatagac a                                                 21

<210>  127

<211>  20

<212>  DNA

<213>  Artificial Sequence

<220>

<223>  primer 5'TAG011GENE-2

<400>  127

gaagagaaag gactcagtgt                                                    20

<210>  128

<211>  20

<212>  DNA

<213>  Artificial Sequence


<220>

<223>  primer 3'TAG011GENE-2

<400>  128
agatatattt acaggatagt                                                    20

<210>  129

<211>  18

<212>  DNA

<213>  Artificial Sequence


<220>

<223>  primer 5'TAG012GENE-2

<400>  129
aaatccaaga ctatgaga                                                      18

<210>  130

<211>  19

<212>  DNA

<213>  Artificial Sequence


<220>

<223>  primer 3'TAG012GENE-2

<400>  130
cttagtggct ggtgacagt                                                     19

<210>  131

<211>  20

<212>  DNA

<213>  Artificial Sequence


<220>

<223>  primer 5'TAG013GENE-2

<400>  131
aacttgccct gtgcctgtgt                                                    20

<210>  132

<211>  20

<212>  DNA

<213>  Artificial Sequence


<220>

<223>  primer 3'TAG013GENE-2

<400>  132
ggtcccttag actttgagca                                                    20

<210>  133

<211>  20

<212>  DNA

<213>  Artificial Sequence


<220>

<223>  primer 5'TAG014GENE-2

<400>  133
cttctgcgag ctgcatctca                                                    20

<210>  134

<211>  19

<212>  DNA

<213>  Artificial Sequence


<220>

<223>  primer 3'TAG014GENE-2

<400>  134
tgcagtgaca gctccgtct                                                     19

<210>  135

<211>  20

<212>  DNA

<213>  Artificial Sequence

<220>

<223>  primer 5'TAG015GENE-2

<400>  135
agaggaggtt tatgtgaaga                                                    20

<210>  136

<211>  21

<212>  DNA

<213>  Artificial Sequence


<220>

<223>  primer 3'TAG015GENE-2

<400>  136
actatctccc aaagaaggac t                                                  21

<210>  137

<211>  18

<212>  DNA

<213>  Artificial Sequence


<220>

<223>  primer 5'TAG016GENE-2

<400>  137
tgtcctcgtc tgcaccat                                                      18

<210>  138

<211>  19

<212>  DNA

<213>  Artificial Sequence


<220>

<223>  primer 3'TAG0G16ENE-2

<400>  138
atgtatttct ggacccact                                                     19

<210>  139

<211>  22

<212>  DNA
<213>  Artificial Sequence

<220>
<223>  primer 5'TAG017GENE-2
<400>  139
gtcaccttct gtgactatgc ct                                                22

<210>  140
<211>  21
<212>  DNA
<213>  Artificial Sequence

<220>
<223>  primer 3'TAG017GENE-2
<400>  140
acaggtcagg cctccatatc t                                                 21

<210>  141
<211>  17
<212>  DNA
<213>  Artificial Sequence

<220>
<223>  primer 5'TAG018GENE-2
<400>  141
cggctggaca cgtcgga                                                      17

<210>  142
<211>  18
<212>  DNA
<213>  Artificial Sequence

<220>
<223>  primer 3'TAG018GENE-2
<400>  142
ggcggaagtg gtggtact                                                     18

<210> 143

<211> 19

<212> DNA

<213> Artificial Sequence

<220>

<223> primer 5'TAG019GENE-2

<400> 143
cacaacctcg actactaca 19

<210> 144

<211> 18

<212> DNA

<213> Artificial Sequence

<220>

<223> primer 3'TAG019GENE-2

<400> 144
gccctccttc agcagctt 18

<210> 145

<211> 23

<212> DNA

<213> Artificial Sequence

<220>

<223> primer 5'TAG022GENE-2

<400> 145
ttcacaaata caccagacgt gat 23

<210> 146

<211> 23

<212> DNA

<213> Artificial Sequence

<220>

<223> primer 3'TAG022GENE-2

<400> 146
gggcgctgct ccatggctct gct 23

<210> 147

<211> 19

<212> DNA

<213> Artificial Sequence


<220>

<223> primer 5'TAG025GENE-2

<400> 147
tgcctagaaa ggggtggct 19

<210> 148

<211> 22

<212> DNA

<213> Artificial Sequence


<220>

<223> primer 3'TAG025GENE-2

<400> 148
ttctcagtgc agacatgtgg ct 22

<210> 149

<211> 23

<212> DNA

<213> Artificial Sequence


<220>

<223> primer 5'TAG029GENE-2

<400> 149
caggcttctg atagtttgca act 23

<210> 150

<211> 19

<212> DNA

<213> Artificial Sequence

<220>

<223>  primer 3'TAG029GENE-2

<400>  150
tatgctattc agagaaact                                                    19

<210>  151

<211>  19

<212>  DNA

<213>  Artificial Sequence


<220>

<223>  primer 5'TAG030GENE-2

<400>  151
tctaatgcat gtagaagct                                                    19

<210>  152

<211>  22

<212>  DNA

<213>  Artificial Sequence


<220>

<223>  primer 3'TAG030GENE-2

<400>  152
agggcagagt cgacaaaaca gt                                                22

<210>  153

<211>  20

<212>  DNA

<213>  Artificial Sequence


<220>

<223>  primer 5'TAG032GENE-2

<400>  153
tcttgagtgg gctagtgact                                                   20

<210>  154

<211>  20

<212>  DNA

<213>  Artificial Sequence

<220>
<223>  primer 3'TAG032GENE-2

<400>  154
agtctggcaa tggagcatga                                                    20

<210>  155
<211>  19
<212>  DNA
<213>  Artificial Sequence

<220>
<223>  primer 5'TAG036GENE-2

<400>  155
tgctatacct tgacttcat                                                     19

<210>  156
<211>  18
<212>  DNA
<213>  Artificial Sequence

<220>
<223>  primer 3'TAG036GENE-2

<400>  156
tccaagtgta ctgcttat                                                      18

<210>  157
<211>  19
<212>  DNA
<213>  Artificial Sequence

<220>
<223>  primer 5'TAG036GENE-2.1

<400>  157
ctagtagtca gttgggagt                                                     19

<210>  158

<211> 19

<212> DNA

<213> Artificial Sequence

<220>

<223> primer 3'TAG036GENE-2.1

<400> 158
agccagaaca gcctttact 19

## Description

The invention relates to the field of medicine. The invention particularly relates to the fields of molecular biology and detection methods.

Recent advances in the knowledge of molecular processes in a cell and techniques to study these processes have resulted in improved methods of typing and treating diseases, Understanding of the underlying molecular diversity of tumors has, for instance, already led to a better understanding of the diversity of response to treatment of morphologically similar tumors. Improved typing influences the way tumor patients are being treated. A drawback of the current methods of treatment is, however, that it takes a relatively long time to determine whether a treatment given to a patient is actually effective. This impedes the optimization of dosages and/or schedules with which treatment is given. Moreover, it also slows down the possibility to adjust the treatment regimen all together. For instance, adjustment of therapy is currently only possible when macroscopic analysis of tumor cells in the body indicates that the therapy given is not effective. Macroscopic changes typically need several weeks to manifest themselves and equipment to measure such changes is often not readily available,

The present invention provides a method for determining whether a treatment is effective in changing a status of a certain set of target cells in an individual comprising obtaining a sample from said individual after initiation of said treatment and determining whether said sample comprises an expression product of at least one marker gene. In one embodiment of the invention said set of target cells comprises a tumor cell. By changing a status of a set of target cells is meant herein that at least one property of said set of target cells is altered. For instance, the amount of said target cells may be changed. Said amount may either be increased or decreased. Alternatively, the activity of said target cells may be altered. Said activity may be a replication activity. As another example, said activity may be an activity involved with angiogenesis. Alternatively, said activity may be an apoptotic activity.

It was found that tumor cells and/or surrounding tissue respond, on a molecular level, very quickly to an effective treatment. This response can be detected by measuring an expression product of a marker gene. Marker gene expression products are indicative for a response to treatment. Marker genes are typically genes that are expressed by said set of target cells, for instance tumor cells, and/or surrounding tissue. However, marker genes can also be expressed in non-tumor target cells in other compartments of the body, for instance blood cells and/or cardiovascular cells,

Alternatively, marker gene expression can be initiated upon treatment given to the individual. Marker gene expression products are responsive to treatment given to a patient. A response can be an alteration in the relative amounts of expression product. However, it can also be an alteration in absolute presence or absence of expressed product such as RNA and/or protein.

According to the invention, a sample which is obtained from a patient may comprise at least one of said target cells. This is particularly suitable for detecting circulating tumor cells which have released themselves from a tumor and are circulating in the blood of a patient. Alternatively, said target cells may be non-tumor cells, In another embodiment of the invention, said sample does not comprise any target cells. However, said sample may comprise another, non-target cell. Expression, or change of expression, of at least one marker gene by said non-target cell is indicative for the status of a certain set of target cells. Said non-target cell preferably comprises a peripheral blood mononuclear cell, as is described below. In yet another embodiment, said sample does not comprise any cell at all. For instance, an expression product of a marker gene, produced by a target cell or non-target cell elsewhere in an individual's body, may be present in said sample at detectable levels.

With a method of the invention it is possible to determine whether a treatment is effective in said individual. This can be done while a treatment is given or shortly after said treatment. Thus it is possible for instance to adjust treatment schedule, dosages and type on a patient per patient basis. It is preferred that said sample is obtained within a week of initiation of treatment. More preferably, said sample is obtained within two days of initiation of treatment. With a method of the invention it is possible to evaluate treatment effectiveness almost immediately after initiation of said treatment. A method of the invention thus offers a good opportunity for determining whether treatment adjustments are required,

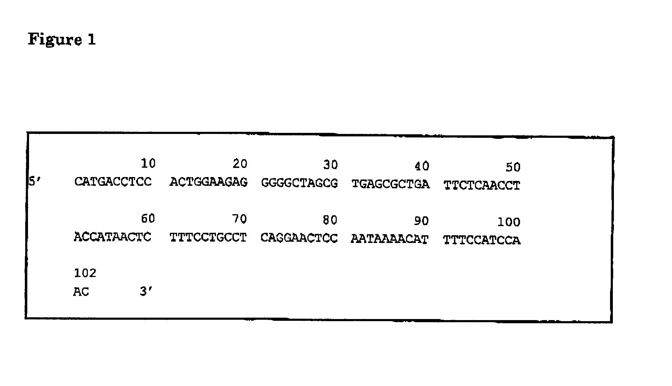

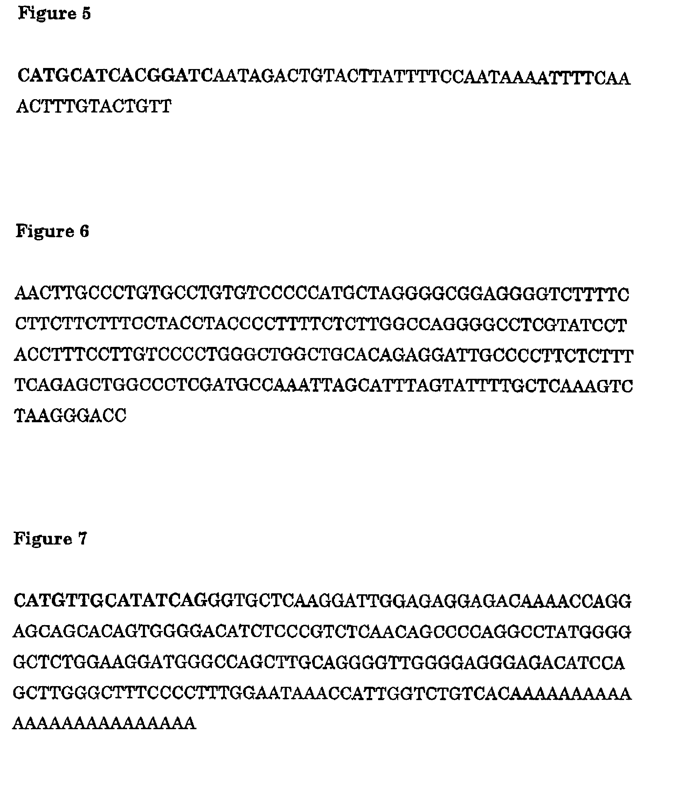

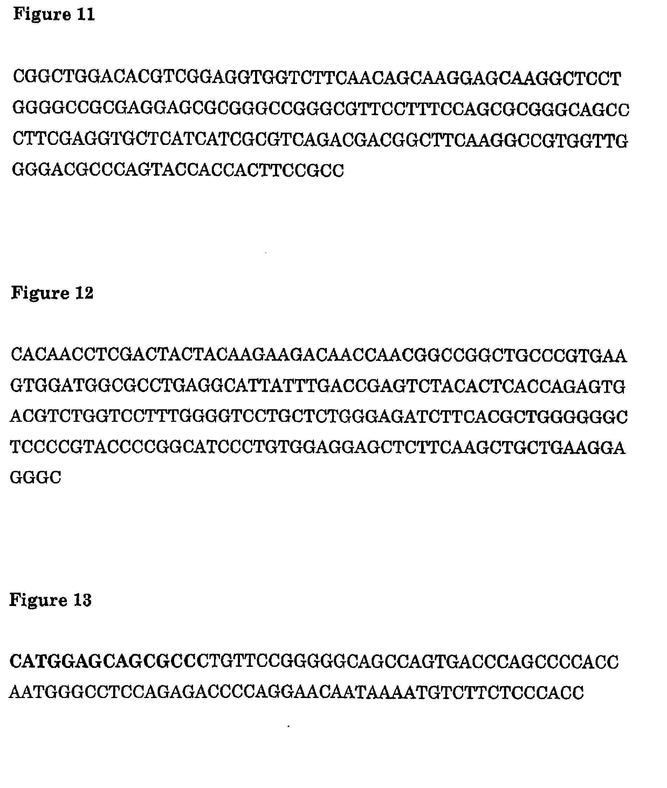

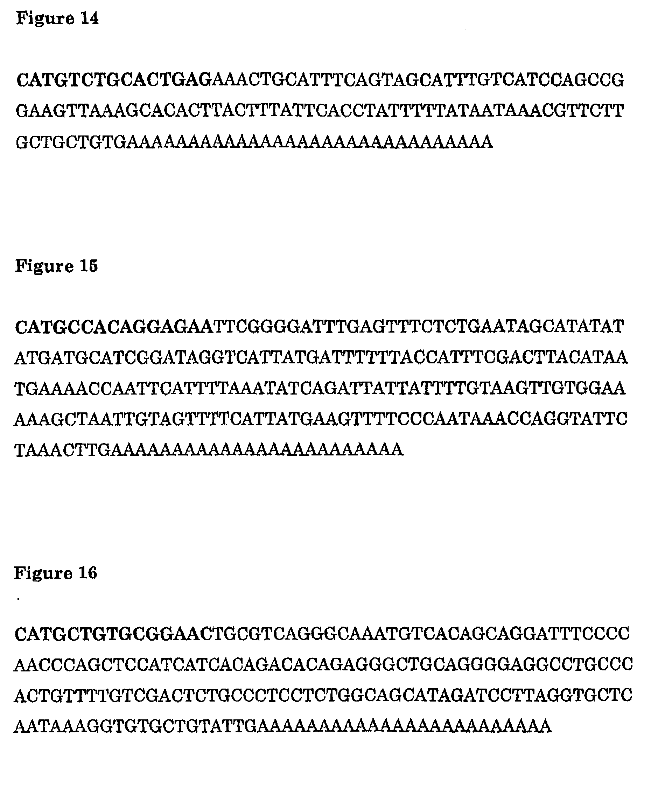

A marker gene preferably comprises a gene involved in the generatíon, maintenance and/or breakdown of blood vessels (angiogenesis). Classes of genes involved in the process of angiogenesis encompass among others receptors, ligands and signaling molecules. Tumor cells are dependent on the growth of new blood vessels to maintain expansion of tumor mass. On the one hand, blood vessels are required to carry nutrients to the site of the tumor, whereas on the other hand waste material needs to be transported from the tumor. In the present invention it has been shown that expression products from genes involved in the generation, maintenance and breakdown of a blood vessel are among the first to respond to anti-tumor treatments. Such genes are therefore very suitable marker genes of the invention. In one embodiment said marker gene comprises a sequence as depicted in table 1 or 2. In another embodiment said marker gene comprises a sequence as depicted in figures 1-18, or a part or analogue thereof,

A change in the level of expression product of a marker gene is indicative for whether a treatment is effective or not. For instance, the level of expression product of a marker gene can be enhanced in a sample when a treatment is effective, alternatively expression product of a marker gene can be reduced. Thus, preferably, expression product of a marker gene is quantified. The level of expression product in a sample can vary due to changes in the expression of a marker gene. However, it is also possible that the level changes due to a change in type of cells comprising said expression product in said sample, for instance due to treatment related cell death at the site of the body where the sample is obtained. Considering that the level of expression product of marker genes can vary from patient to patient, it is preferred that a method of the invention further comprises comparing the level of expression product of said marker gene with a reference. Preferably said reference comprises the same type of tumor cells prior to, or in the absence of, said treatment. Preferably, said tumor cells are derived from the same patient. The difference in the level of expression product of a marker gene in an effective and a non-effective treatment can be very large. In the extreme cases the level of expression product can range from detectable to not detectable. Marker genes displaying such zero to one relation in expression product levels are preferred in the present invention. A zero to one relation can be used to design relatively simple test systems. A zero to one relation is of course dependent on the detection system used to detect expression product of a marker gene. Very sensitive expression detection systems will typically detect expression product where a less sensitive systems detects no expression product. An expression product can be RNA or a part thereof, transcribed from said marker gene or a translated protein or a part thereof. A person skilled in the art is well capable of designing the most appropriate expression detection system to practice this preferred embodiment of the invention.

A part of an RNA or DNA molecule is defined herein as an RNA or DNA sequence, comprising at least 50 nucleotides. A part and/or an analogue of an expression product is defined herein as a part and/or analogue that can be detected using essentially the same kind of detection method as said expression product, although the sensibility of detection may differ. An analogue of an RNA or DNA molecule is defined herein as an RNA or DNA sequence which is essentially the same as a particular RNA or DNA sequence. However, a nucleotide mutation, replacement, alteration, addition and/or deletion may have taken place naturally and/or performed artificially, without essentially altering the detection of said analogue as compared with the detection of said particular RNA or DNA sequence. A person skilled in the art is well able to determine whether a given RNA or DNA sequence is an analogue of a particular RNA or DNA sequence, using techniques known in the art.

In a preferred embodiment said tumor comprises Kaposi's Sarcoma. Kaposi's Sarcoma is a disease of proliferating blood vessels and therefore very much suited for identifying marker genes involved in angiogenesis. According to the invention, changes in angiogenesis factors are among the first marker events as a result of treatment, Kaposi's Sarcoma (KS) manifests itself clinically by reddish skin lesions. Kaposi's Sarcoma is a multicentric, malignant neoplastic vascular proliferation characterized by the development of bluish-red cutaneous nodules, usually on the lower extremities, most often on the toes or feet, and slowly increasing in size and number and spreading to more proximal areas. The tumors have endothelium-lined channels and vascular spaces admixed with variably sized aggregates of spindle-shaped cells, and often remain confined to the skin and subcutaneous tissue, but widespread visceral involvement may occur. Kaposi's Sarcoma occurs spontaneously in Jewish and Italian males in Europe and the United States. An aggressive variant in young children is endemic in some areas of Africa. A third form occurs in about 0.04% of kidney transplant patients. There is also a high incidence in AIDS patients. (From Dorland, 27th ed & Holland et al., Cancer Medicine, 3d ed, pp2105-7)

Kaposi's Sarcoma is aggressive in HIV infected individuals. The angiogenic mechanism causing the lesions results from the interplay of viral and cellular gene expression and is poorly understood in terms as to which genes are involved and what controls their expression. The angiogenic proliferation in KS involves mechanisms likely to be universal in angiogenesis. The central role of angiogenesis in Kaposi's Sarcoma is clearly illustrated by the French name for this tumor: angiosarcomatose kaposi. Because of said central role of angiogenesis in Kaposi's Sarcoma, determination of marker genes involved in angiogenesis is very suitable to determine whether a treatment of Kaposi's Sarcoma is effective.

In the present invention, gene expression patterns of Kaposi's Sarcoma were examined with a method called serial analysis of gene expression (SAGE) (Velculescu et al. (1995) Science 270; 484-487). This method allows the quantitative and simultaneous analysis of a large number of transcripts. SAGE is based on two principles. First, a short nucleotide sequence TAG (14 base pairs) contains sufficient information to uniquely identify a transcript, provided it is isolated from a defined position within the transcript. Second, concatenation of short sequence TAG's allows the efficient analysis of transcript in a serial manner by sequencing of multiple TAG's within a single clone.

Briefly, in this method a biotinylated oligo (dT) primer is used to synthesize cDNA from mRNA, and after digestion with a restriction enzyme, the most 3' terminus (near the poly-A tail) is isolated. These 3' fragments of cDNA are ligated to linkers and cleaved with a type II restriction enzyme to release short sequence (14 bp) of the original cDNA (TAG's). The TAG's are ligated to diTAG's and PCR amplified. These di-TAG's are then ligated to form long concatamers, which are cloned and sequenced. In this way, one sequence reaction yields information about the distribution of many different mRNA's. Finally, the calculation of the abundance of different TAG's and the matching of the TAG's in Genbank are done using the necessary computer software.

In another aspect the invention provides the use of a nucleic acid comprising a sequence as depicted in figure 1-18 and/or table 1 or 2, or a part or analogue thereof, in an expression product detection method. Expression of a marker gene in an individual can be detected by determining whether said nucleic acid or part or analogue is able to hybridize with nucleic acid, preferably RNA, in a sample of said individual. If hybridisation takes place, it is indicative of expression of a marker gene in said individual. Of course, as is known by a person skilled in the art, a coding strand of DNA/RNA is capable of hybridizing with the complementary strand of a corresponding doublestranded nucleic acid sequence. Hence, a complementary strand of a certain coding strand is particularly suitable for detection of expression of said coding strand. For instance, a complementary strand of a coding strand as depicted in figure 1-18 and/or table 1 or 2 is suitable for detection of expression of a gene comprising said coding strand.

In yet another aspect, the invention provides the use of a proteinaceous molecule capable of specifically binding a protein encoded by a nucleic acid comprising a sequence as depicted in figure 1-18 and/or table 1 or 2, or a part or analogue thereof, in a detection method. In one embodiment of the invention, said uses are directed toward determining the presence of a site of angiogenesis in an individual. In another embodiment of the invention, said uses are directed toward determining the presence of a tumor cell in an individual. The presence of a tumor cell in an individual can be determined because said tumor cell typically expresses marker genes that can be detected by an expression product detection method. For instance, an antibody, or analogue thereof, specifically directed against an expression product of said marker gene can be generated. Said antibody or analogue is suitable for determination of an expression product of said marker gene in a sample. To determine the presence of a tumor cell in an individual, a sample from said individual can be incubated with said antibody. If said sample contains an expression product of said marker gene, said antibody will bind. Binding can be demonstrated by techniques known in the art, like for instance ELISA. If binding of said antibody is demonstrated, one can conclude that said sample contains an expression product of said marker molecule. The presence of an expression product of said marker molecule can indicate the presence of a tumor cell in an individual, since said marker molecule is expressed by tumor cells. There are of course many more alternative techniques to detect an expression product with use of a proteinaceous binding molecule, which are well known in the art and need no further discussion here. Thus, proteins expressed by a tumor cell can be detected by a proteinaceous molecule capable of specifically binding a protein encoded by a nucleic acid comprising a sequence as depicted in figure 1-18 and/or table 1 or 2, or a part or analogue thereof. Likewise, the presence of a site of angiogenesis in an individual can be determined by detecting an expression product of a marker gene.

In another embodiment, a use of a nucleic acid comprising a sequence as depicted in figure 1-18 and/or table 1 or 2, or a use of a proteinaceous molecule capable of specifically binding a protein encoded by a nucleic acid comprising a sequence as depicted in figure 1-18 and/or table 1 or 2, or a part or analogue thereof, are directed toward determining whether a treatment is effective in changing the status of a certain set of target cells in an individual. More preferably, said uses are directed toward determining whether a treatment is effective in counteracting a tumor in an individual. In one embodiment of the invention, said tumor comprises Kaposi's Sarcoma.

In one aspect the invention provides the use of a nucleic acid comprising a sequence as depicted in table 1 or 2 and/or figure 1-18 as an indicator for angiogenesis. For instance, a nucleic acid comprising a sequence as depicted in figure 1-18 and/or table 1 or 2 can be used as detection marker for the process of angiogenesis in the course of regenerative treatment. Changes in the expression level of the detection marker indicate active growth of blood vessels (i.e. angiogenesis) as was meant to induce with the regenerative treatment course. In a preferred embodiment such application is in the field of heart and coronary disease aimed at generation of new blood supply to affected organs by means of new blood vessels. Likewise, the treatment of tumors with anti-angiogenesis drugs can be monitored by changes in expression levels of detection marker genes as depicted in figure 1-18 and/or table 1 or 2.

In another aspect the invention provides the use of a nucleic acid comprising a sequence as depicted in figure 1-18 and/or table 1 or 2 as detection marker for tumor cells. In yet another aspect the invention provides the use of a proteinaceous molecule encoded by a nucleic acid comprising a sequence as depicted in figure 1-18 and/or table 1 or 2 or a proteinaceous molecule capable of binding a protein encoded by a nucleic acid comprising a sequence as depicted in figure 1-18 and/or table 1 or 2 as detection marker for tumor cells.

With a method of the invention it is possible to monitor a specific status of an individual. The presence of a disease - or danger of developing one - can be determined by determining whether or not a sample of an individual comprises an expression product of a marker gene. This is possible for any disease, as long as the disease involves an altered expression pattern of at least one marker gene. Additionally, a healing process can be followed as well. For instance, recovery of damaged tissue can involve an increasing amount of expression product of a marker gene over time. It is however also possible that recovery of damaged tissue involves a decreasing amount of expression product of a marker gene over time. Samples taken at different time intervals provide information about the amount of expression product which is generated at different time points. An altered amount of a specific expression product found in samples during a period of time is indicative of the amount of tissue cells generated. Likewise, a decreasing amount of an expression product found in samples in a specific time-period can indicate a certain - either beneficial or harmful - process. For instance, said process may involve the development or the treatment of disease. An important application is a treatment of heart and coronary disease. A method of the invention is very suitable for monitoring the generation of new cardiac tissue.

Thus, one aspect of the invention provides a method of diagnosis, in particular a method for determining whether an individual comprises a tumor cell and/or a site of angiogenesis, comprising obtaining a sample from an individual, and determining whether said sample comprises an expression product of at least one marker gene. Preferably, said marker gene comprises a sequence as depicted in table 1 or 2 and/or figure 1-18, or a part or analogue thereof. With a method of the invention it is possible to detect tumor cells that have released themselves from the tumor and are elsewhere in the body. In a preferred embodiment such detection is performed in the blood of a person detecting circulating tumor cells. These circulating tumor cells can be used for primary identification of presence of a tumor somewhere in the body and also for identification of the risk of metastasis of the tumor to other places in the body next to the primary location of the body. Likewise, a method of the invention is suitable for determining a site of angiogenesis in an individual. Angiogenesis is an indicator for different aspects. For instance, an increased level of angiogenesis indicates the presence of tumor cells, or the healing of damaged tissue, like for instance recovery of heart and coronary disease.

Since an angiogenic process is now easily monitored by a method of the invention, it is likewise easy to determine whether a certain treatment is effective in altering an angiogenic process. For instance, if a certain treatment is effective in counteracting an angiogenic process, the amount of an expression product of a marker gene involved in angiogenesis decreases as well over time. In the art, many drugs are known for anti-angiogenic treatment. Thus, one embodiment of the invention provides a method for determining whether a treatment is effective in altering an angiogenic process in an individual comprising obtaining a sample from said individual after initiation of said treatment, and determining whether said sample comprises an expression product of at least one marker gene. Preferably, said marker gene comprises a sequence as depicted in table 1, 2, and/or figures 1-18, or a part or analogue thereof. In one embodiment, said treatment comprises counteracting angiogenesis in said individual. In yet another embodiment said treatment involves the use of at least one of the following drugs: 2ME2, Angiostatin, Angiozyme, Anti-VEGF RhuMAb, Apra (CT-2584), Avicine, Benefin, BMS275291, Carboxyamidotriazole, CC4047, CC5013, CC7085, CDC801, CGP-41251 (PKC 412), CM101, Combretastatin A-4 Prodrug, EMD 121974, Endostatin, Flavopiridol, Genistein (GCP), Green Tea Extract, IM-862, ImmTher, Interferon alpha, Interleukin-12, Iressa (ZD1839), Marimastat, Metastat (Col-3), Neovastat, Octreotide, Paclitaxel, Penicillamine, Photofrin, Photopoint, PI-88, Prinomastat (AG-3340), PTK787 (ZK22584), RO317453, Solimastat, Squalamine, SU 101, SU 5416, SU-6668, Suradista (FCE 26644), Suramin (Metaret), Tetrathiomolybdate, Thalidomide, TNP-470, and/or Vitaxin. However, the artisan can think of more drugs that can be used during said treatment,

In a preferred embodiment, a sample of a method of the invention is a blood sample. Although the location of for instance an angiogenic process can be a tumor or a part of the skin, a blood sample is preferred, among other things because it is much easier to obtain. A blood sample is also often easier to investigate, requiring less expensive and/or specific equipment. Quite surprisingly, we have found that the expression of certain marker genes by hemopoietic cells, like peripheral blood mononuclear cells (PBMC), can be indicative for a process occurring somewhere else in an individual's body. For instance, the presence, or alteration in amount, of an expression product of a marker molecule in PBMC can indicate the presence of a tumor somewhere in the body. In example 10 it is for instance shown that a TIE 1 sequence (tag 15, table 3) or a Salioadhesin or Siglec 1 sequence (tag 32, table 4) are both upregulated in skin tumor and in PBMC cells in a Kaposi's Sarcoma patient. Additionaly, example 8 shows that the absence of expression product of a Keratin 14 sequence (tag 7, table 3) in a blood sample of said patient, whereas Keratin 14 is overexpressed in tumor cells, indicates that said sample was not contaminated with tumor cells. Likewise, the expression of certain marker genes by PBMC can provide another diagnostic indication. The presence or absence of an expression product of a marker gene in PBMC provides adequate information about different aspects and/or processes of an individual's body. Preferably, the amount of expression product in a non-hemopoieteic cell is compared with a reference value. This way an indication is obtained about an increment or decrement of expression in said hemopoietic cell.

In one aspect the invention therefore provides a method for determining whether an individual comprises a non-hemopoietic tumor cell and/or a site of angiogenesis, said method comprising determining whether a hemopoietic cell from said patient comprises an altered amount of an expression product of a marker gene as compared with a reference value. Preferably, said marker gene comprises a gene involved in angiogenesis. More preferably, said gene comprises a sequence as depicted in table 1 or 2, and/or figures 1-18, or a part or analogue thereof. In one embodiment of the invention said hemopoietic cell comprises a peripheral blood mononuclear cell.

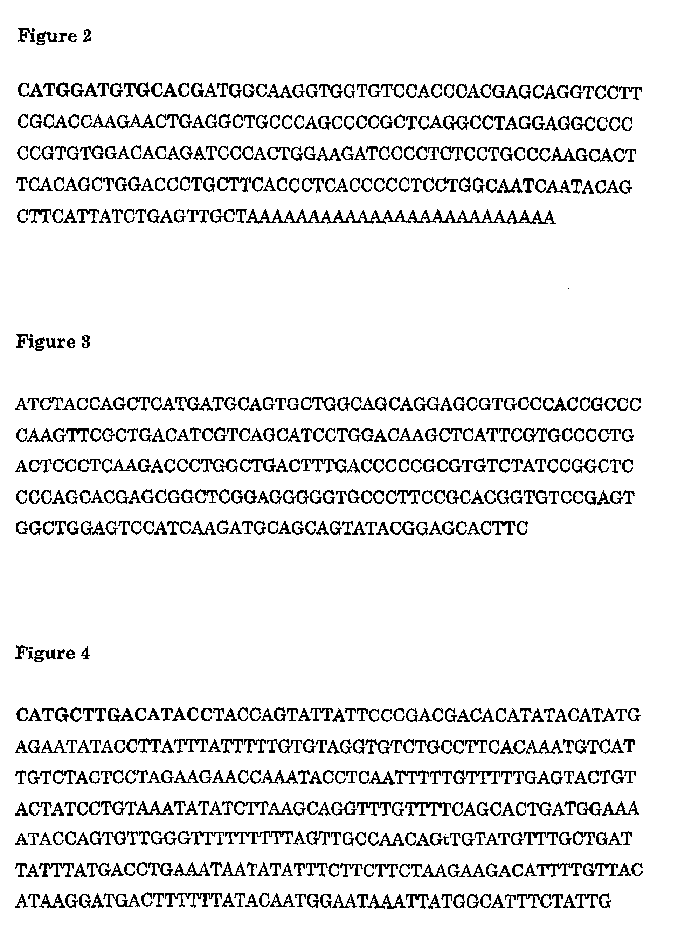

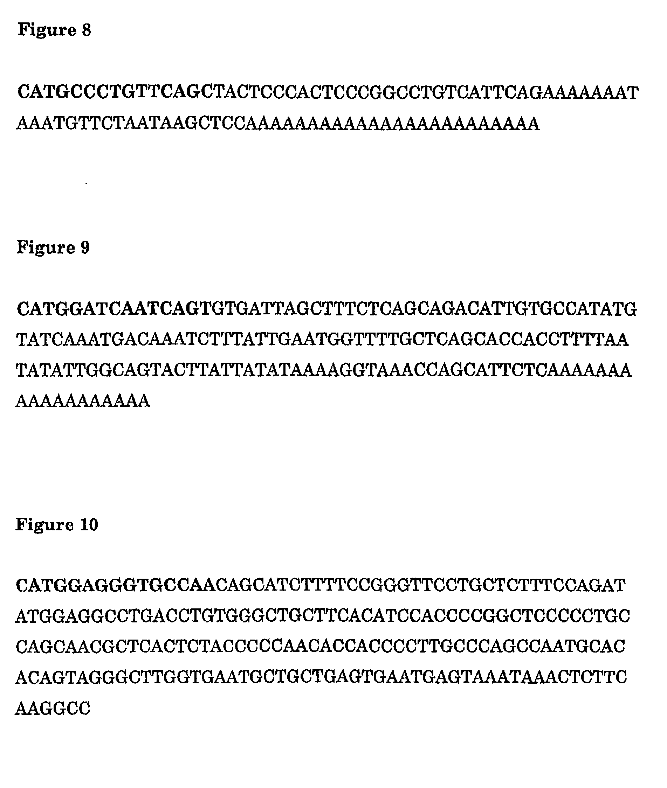

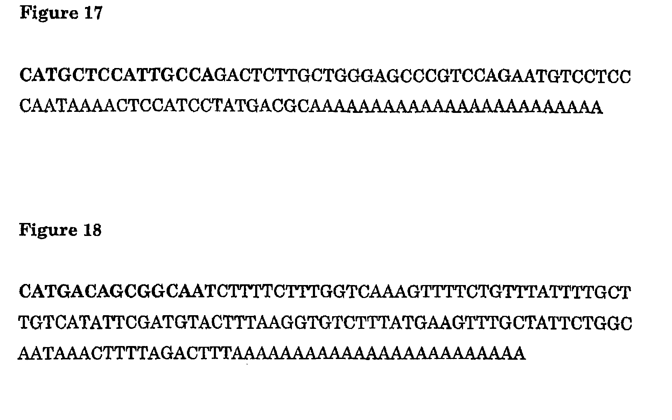

In one aspect the invention provides a method of the invention, wherein said expression product is expressed by a PBMC. Preferably, said expression product comprises a TIE 1 sequence, a Salioadhesin or Siglec 1 sequence, a sequence as depicted in figure 8 or 17, or a part or analogue thereof. As these sequences are involved in angiogenesis, the invention also provides a use of a PBMC expressed Keratin 14 sequence, TIE 1 sequence, Salioadhesin or Siglec 1 sequence, a sequence as depicted in figure 2, 8 or 17, or a part or analogue thereof, as an indicator for angiogenesis. Likewise, these sequences are involved in the presence of tumor cells. Therefore, a use of a PBMC expressed Keratin 14 sequence, TIE 1 sequence, Salioadhesin or Siglec 1 sequence, a sequence as depicted in figure 2, 8, or 17, or a part or analogue thereof, for determining the presence of a tumor cell in an individual, is also herewith provided. Additionally, the invention also provides an isolated Keratin 14 sequence, TIE 1 sequence, Salioadhesin or Siglec 1 sequence, a sequence as depicted in figure 2, 8, or 17, or a part or analogue thereof, for use in a diagnostic method. A diagnostic method can be carried out using a diagnostic kit. Therefore, a diagnostic kit comprising a nucleic acid comprising a sequence as depicted in figure 1-18 and/or table 1 or 2, or a part or analogue thereof, and/or a proteinaceous molecule capable of specifically binding a protein encoded by said nucleic acid or said part or analogue, is also herewith provided. Preferably, said kit comprises a suitable means of detection. In one embodiment a diagnostic kit of the invention is provided comprising a Keratin 14 sequence, and/or a TIE 1 sequence, and/or a Salioadhesin or Siglec 1 sequence, and/or a sequence as depicted in figure 2, 8 or 17, or a part or analogue thereof.
A diagnostic kit of the invention is particularly useful for carrying out a method of the invention. In yet another embodiment the invention therefore provides a use of a diagnostic kit of the invention for determining whether a treatment is effective in changing the status of a certain set of target cells in an individual and/or altering an angiogenic process in an individual.
Additionally, the invention provides a use of a diagnostic kit of the invention for determining whether an individual comprises a tumor cell and/or a site of angiogenesis.

With a marker gene of the invention it is possible to screen for drugs directed against a disease for which said marker gene is indicative. There are many methods available in the art for screening for a specific drug activity.
For instance, cells can be incubated with different potential drug compounds, and an expression pattern of a marker gene in said cells before and after exposure to each potential drug compound can be compared. A specific difference in an expression pattern after exposure to a particular potential drug compound shows that said compound is a suitable candidate for the development of a medicament. The invention therefore provides in one embodiment a use of an expression product of a gene comprising a sequence as depicted in figure 1-18, table 1 or table 2 as a drugtarget. Preferably, said sequence is a Salioadhesin or Siglec 1, TIE 1, and/or Keratin 14 sequence. A compound capable of altering the activity of Salioadhesin or Siglec 1, TIE 1, Keratin 14, and/or the expression of Salioadhesin or Siglec 1, TIE 1, and/or Keratin 14 in a cell is also herewith provided. Said compound is particularly suitable for the preparation of a medicament.

The present invention is further explained in more detail by the following examples, which do not limit the invention in any way.

### Examples

### Example 1

In this example a selection of samples for analysis of expression profiles is made.
A 31-year old man was demonstrated to be HIV-1 seropositive in February 1997. The initial CD4 cell count was 25 x 10⁶/l. The patient presented within two months a mucocutanous Herpes simplex infection and an extrapulmonary Cryptococcosis for which specific medication was given. The HIV-1 RNA load at presentation was 15,000 copies/ml and increased to 33,000 copies/ml in three months. Then antiretroviral therapy was started with zidovudine, lamivudine and indinavir, Immediately after start therapy the HIV-1 RNA load dropped below detection limit. In November 1997 the patient presented with gradual appearance of an increasing number of violaceous skin lesions that clinically resembled Kaposi's Sarcoma. The diagnosis was confirmed by histological examination of one of the lesions. At start of the chemotherapy (bleomycin, viricristine and adriamycine intravenously) KS had progressed to about 150 cutaneous lesions. The interval between the courses of chemotherapy was three weeks and stopped after the fifth course. Several lesions had disappeared by three weeks of therapy and complete remission was gradually reached after one year.
During chemotherapy several biopsies were taken. The first biopsy was obtained 24 hours after start chemotherapy (named KS1) and the second biopsy after 48 hours (named KS2). All biopsies were flash-frozen in liquid nitrogen immediately after surgical removal and stored at -80°C. Diagnosis of Kaposi's Sarcoma was confirmed histopathologically.
Control SAGE libraries KS3 and KS4 were made from frozen material taken at autopsy from two AIDS patients with Kaposi's Sarcoma, both of which died in 1986 without having had any form of chemotherapy or retroviral treatment.

### Example 2

The expression profiles of the biopsy samples were determined using the SAGE technology. All biopsies were cut with a microtome in 16-20 µm sections and transferred to a tube containing TRIzol. RNA isolation with TRIzol was performed according to the manufacturer's instructions. Poly (A) RNA was obtained using the Micro-FastTrack™ 2.0 mRNA Isolation Kit. cDNA preparation and the subsequent steps were performed as described by Velculescu. Primary analysis of the sequence results was performed using software especially designed for SAGE by the Bioinformatics Laboratory of the Academic Medical Centre, Amsterdam (van Kampen *et al.* USAGE: a web-based approach towards the analysis of SAGE data. Bioinformatics, *in press).* The libraries were also analysed using the Human Transcriptome Map (HTM), a program developed in the AMC, which maps TAG's onto human chromosomes (Caron *et al*. The Human Transcriptome Map reveals a clustering of highly expressed genes in chromosomal domains. Submitted for publication).

We sequenced ∼ 47,000 TAG's from the four biopsies, 47,298 TAG's from KS 1 library, 46,671 from the KS 2 library, 49,335 TAG's from the KS3 library, and 48,814 TAG's from the KS4 library. TAG lists (i.e. individual TAG's plus the number of appearance) were compared with each other in USAGE, TAG sequences with the highest counts were identified with the amct2g database available in USAGE (which is an improved TAG identification compared with the SAGEmap database from CGAP (available from GenBank). Secondly, TAG lists were mapped to chromosome locations with the HTM program, and at the same time compared with specific TAG lists (e.g. vascular endothelium, publicly available), and with a compilation of all TAG lists in the SAGEmap database (designated "All" in HTM) TAG'S belonging to genes specifically up regulated in KS3 and KS 4 identified (Table 1). Nucleotide 15 was determined from the original diTAG list in USAGE. The TAG sequence of 15 nt. was checked with GenBank (BLAST) to confirm its identification. A few TAG's were eliminated because of ambiguity in the 15^{th} nucleotide, or because of misidentification.

### Example 3

Result of the analysis showing the identifiable TAG's derived from known genes with increased expression in Kaposi's Sarcoma SAGE libraries KS3 and KS4 compared to libraries KS1 and KS2. The TAG numbers are first normalized to a level of 100.000 total TAG's sequenced per library to allow comparison between the libraries and comparison with other libraries in the public domain.
The sequence catg precedes each TAG sequence given in column 2 of table 1.

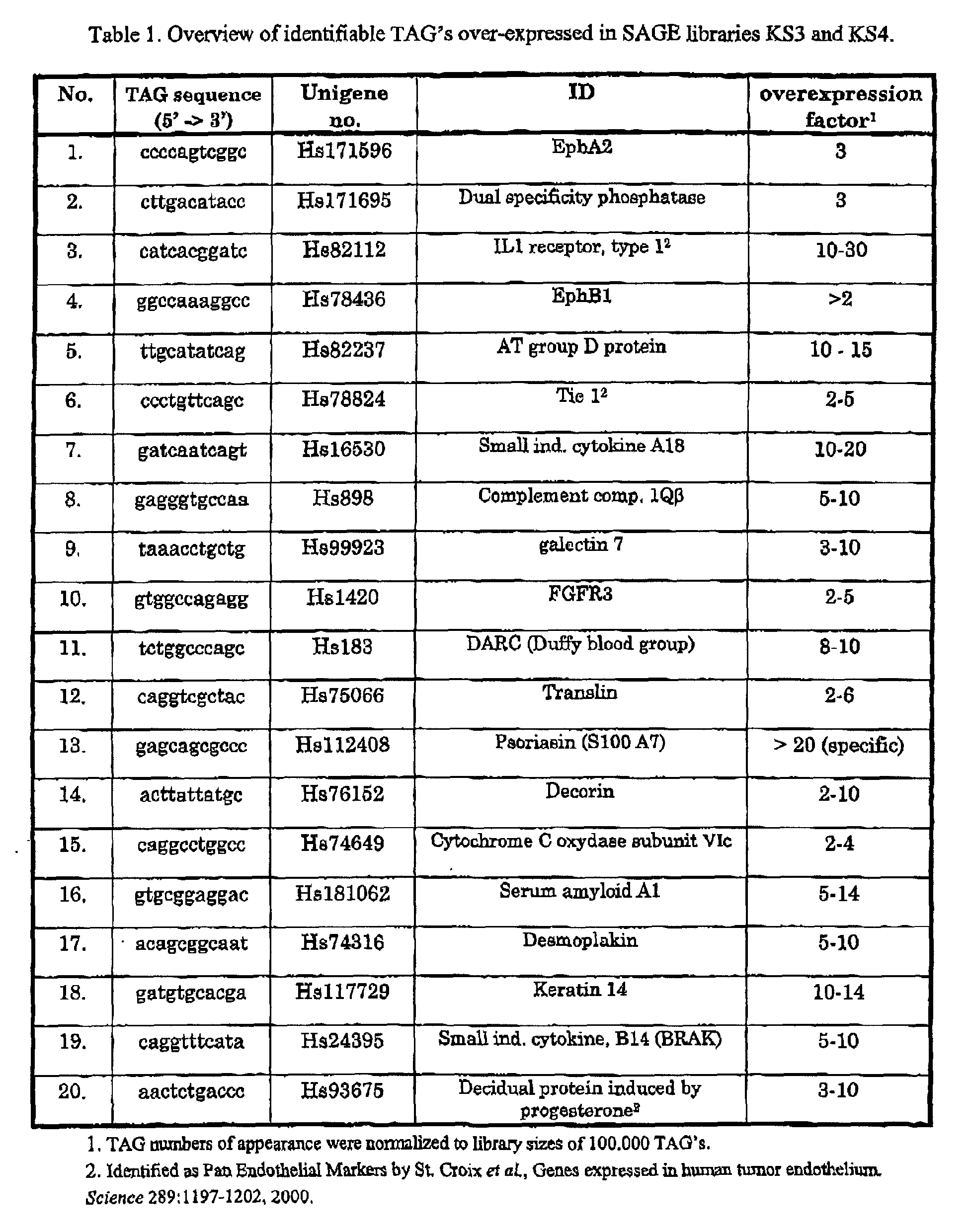

### Example 4

Result of the analysis showing the non-identifiable TAG's derived from EST's of genes with unknown function with increased expression in Kaposi's Sarcoma SAGE libraries KS3 and KS4 compared to libraries KS1 and KS2. The TAG numbers are first normalized to a level of 100.000 total TAG's sequenced per library to allow comparison between the libraries and comparison with other libraries in the public domain.
The sequence catg precedes each TAG sequence given in column 2 of table 2.

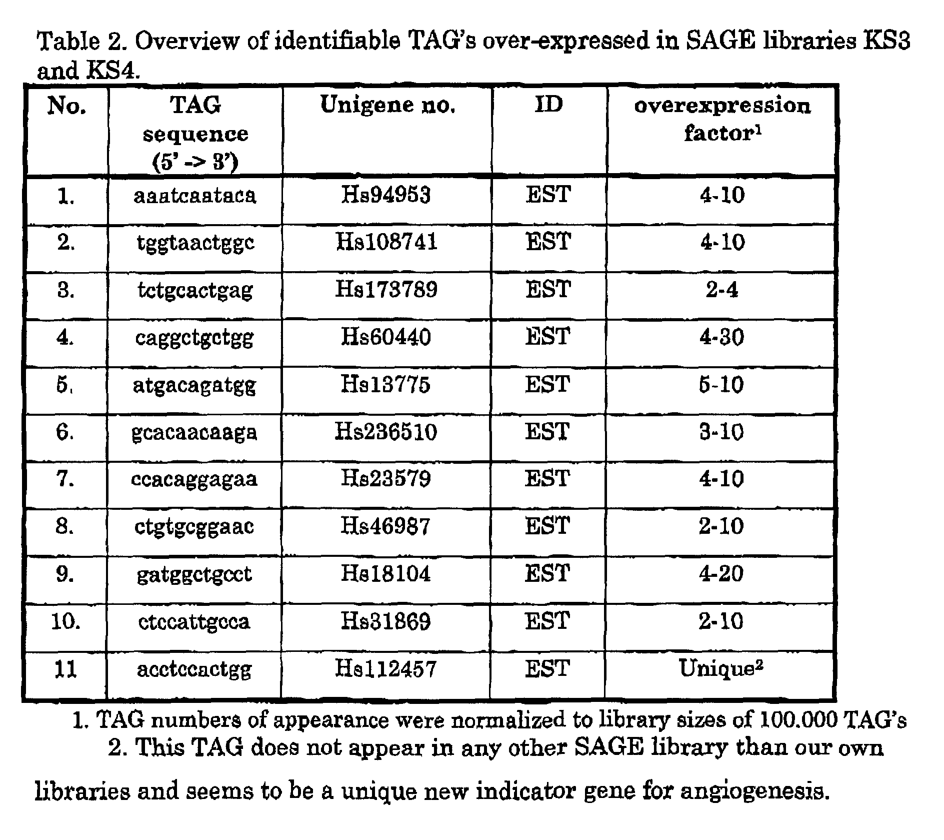

### Example 5

Kaposi's sarcoma skin tissue was obtained from the same two AIDS patients mentioned in example 1 from whom SAGE libraries KS3 and KS4 were made. Both patients were homosexual men and were infected at the beginning of the HIV-1 epidemic in Europe. Patient 1 born in Indonesia was demonstrated to be HIV-1 positive in 1982. In February 1985 a histological examination confirmed the diagnosis of Kaposi's sarcoma. He died 13 month later and postmortem examination revealed morphological variants of visceral KS. Patient 2 presented in February 1984 at the Academic Medical Centre with progressive KS skin lesions. During follow-up the KS progressed to the intestines, oropharynx, lung, tongue, sinus piriformis and lymph nodes. In March 1986 the patient died and autopsy took place. The biopsies of said two patients were named KS3 and KS4.

Normal adult breast skin tissue was obtained as discarded tissue from reduction mammoplasties (obtained from the department of plastic Surgery of our hospital). RNA isolated of three breast reductions was used to construct the normal skin expression profile library.

The expression profiles of the biopsy samples were determined using the SAGE technology as described in example 2.
We sequenced - 47,000 TAG's from the four biopsies, 49,335 TAG's from the KS3 library, and 48,814 TAG's from the KS4 library. TAG lists (i.e. individual TAG's plus the number of appearance) were compared with each other in USAGE, TAG sequences with the highest counts were identified with the amct2g database available in USAGE (which is an improved TAG identification compared with the SAGEmap database from CGAP (available from GenBank). Secondly, TAG lists were mapped to chromosome locations with the HTM program, and at the same time compared with specific TAG lists (e.g, vascular endothelium, publicly available), and with a compilation of all TAG lists in the SAGEmap database (designated "All" in HTM) TAG's belonging to genes specifically up regulated in KS3 and KS 4 identified (Table 1). Nucleotide 15 was determined from the original diTAG list in USAGE. The TAG sequence of 15 nt, was checked with GenBank (BLAST) to confirm its identification. A few TAG's were eliminated because of ambiguity in the 15^{th} nucleotide, or because of misidentification.

### Example 6

Result of the analysis showing the identifiable TAG's derived from known genes with increased expression in Kaposi's Sarcoma SAGE libraries KS3 and KS4 compared to the public libraries of National Center for Biotechnology Information. The TAG numbers are first normalized to a level of 100.000 total TAG's sequenced per library to allow comparison between the libraries and comparison with other libraries in the public domain.
The sequence catg precedes each TAG sequence given in column 2 of table 3.

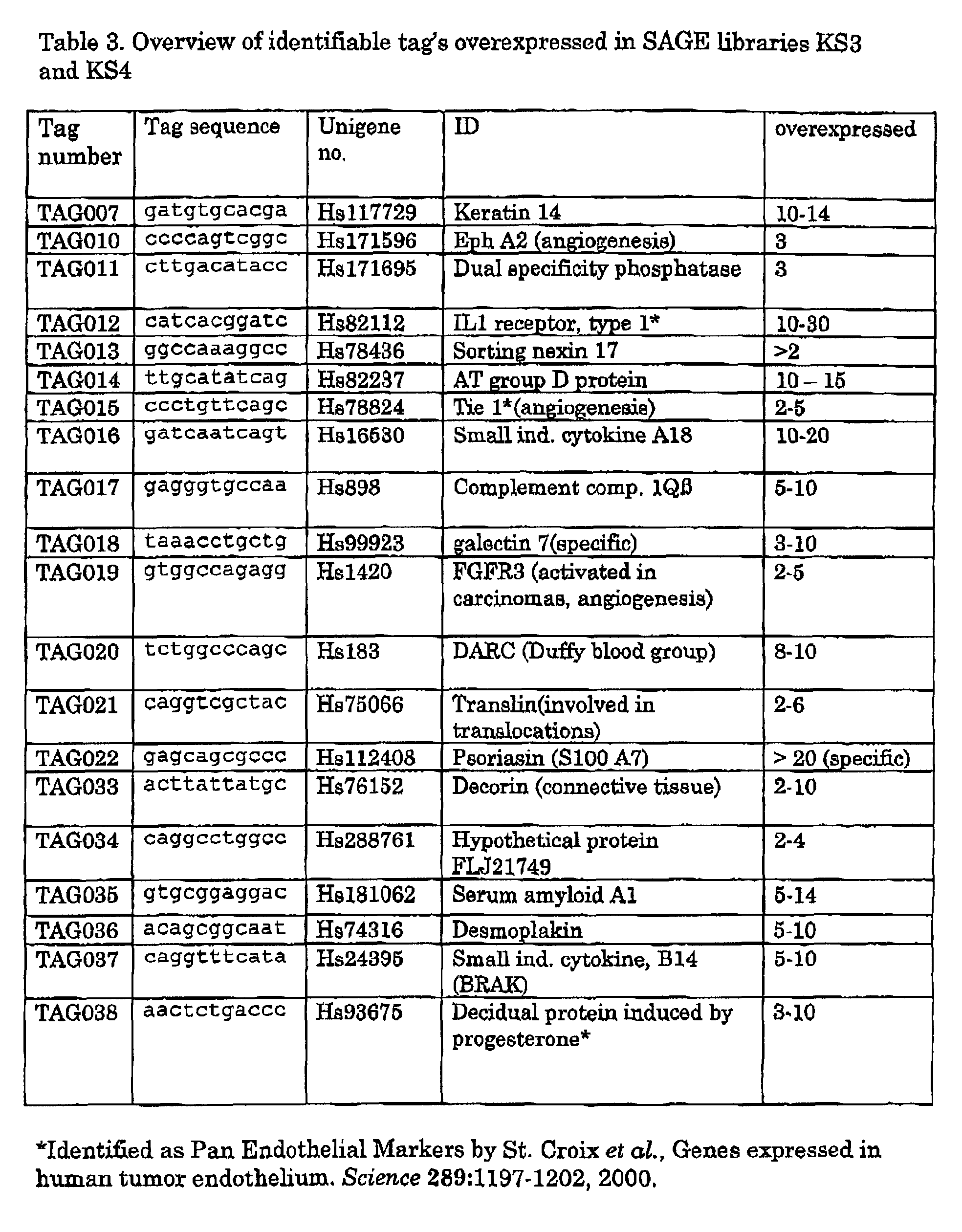

### Example 7

Result of the analysis showing the non-identifiable TAG's derived from EST's of genes with unknown function with increased expression in Kaposi's Sarcoma SAGE libraries KS3 and KS4 compared to libraries KS1 and KS2. The TAG numbers are first normalized to a level of 100.000 total TAG's sequenced per library to allow comparison between the libraries and comparison with other libraries in the public domain.
The sequence catg precedes each TAG sequence given in column 2 of table 4.

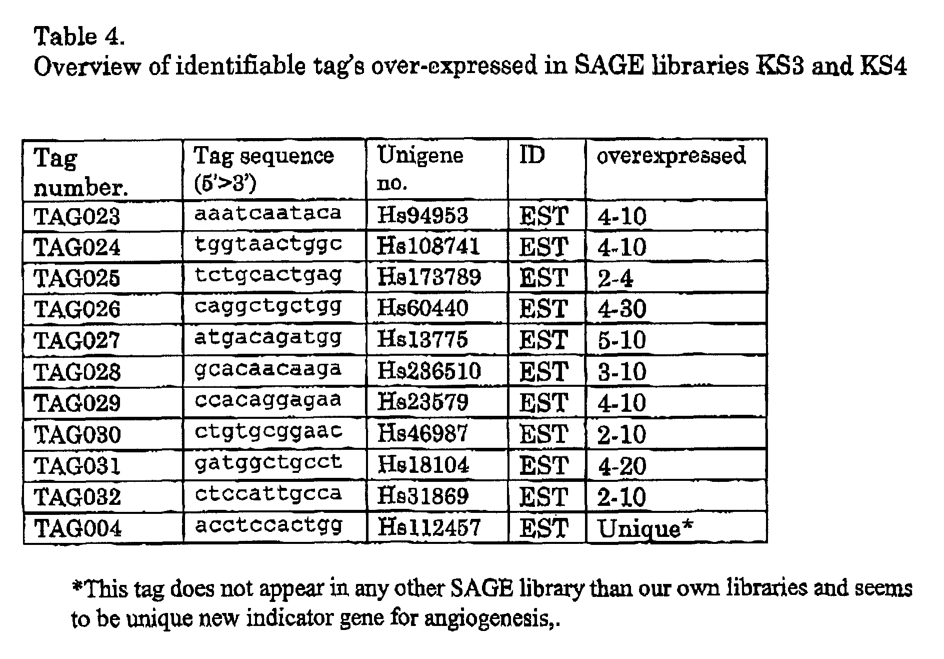

The overexpressed TAG's listed in tables 3 and 4 are the same as in tables 1 and 2, respectively. This shows that the expression pattern after treatment is comparable with the expression pattern of healthy individuals with normal expression patterns.

### Example 8

Using an RT-PCR based method we were able to determine that TAG 11 (table 2) / TAG 004 (table 4) indeed represents a differently expressed gene. RNA was isolated from a KS lesion and the first strand cDNA synthesis was primed with an oligo(dT) primer with a 5' M13 tail (5'CTA GTT GTA AAA CGA CGG CCA G-(T)₂₄ 3'). Ten microliter total RNA was used, plus primer and 5 µl RT-mix (50 mM Tris, pH 8.3, 75mM KCl, 3 mM MgCl₂, 10 mM DTT), 80 mM dNTPs and 20 units RNAsin were added, followed by an incubation for 3 minutes at 65°C and chilled on ice. The RT reaction starts by adding 5 units AMV RT followed by an incubation of 45 minutes at 42°C. For the PCR we used a 19-base TAG-specific primer (which consisted of 11 nt identified in the sage with a 5' NLAIII restriction site and 5 inosine nucleotides to increase the annealing temperature of the primers) and the -21M13 primer, The RT-mix was added to 80 µl PCR mixture containing the 100 ng of each primers (-21m13 PRIMER: 5' GTA AAA CGA CGG CCA GT 3' and 5' III IIC ATG ACC TCC ACT GG 3'), 50mM Tris (pH8.3), 20 mM KCl, 0.1 mg BSA per ml, dNTPs (0.1 mM each), 2,4 mM MgCl₂, and 2 units Taq polymerase. After incubation of 5 minutes at 94°C, the reaction was subjected to 35 cycles of amplification in a thermocycler (9700 Perkin-Elmer). A cycle included denaturation for 1 minute at 95°C, annealing for 1 minute at 55°C and extension for 2 minutes at 72°C. The last cycle was followed by 72°C incubation for 10 minutes.

The amplified fragment was cloned in to an AT plasmid (InvitroGen) and subsequently the insert was sequenced using the dye terminator sequencing kit from Applied Biosystems Inc. The fragment appeared to have a length of 102 base pairs and the sequence analysis of the fragment revealed the sequence as depicted in figure 1. This sequence was identical to an EST sequence identified from human foetal heart (GenBank acc. # AI217565 and others), which in turn matched a predicted exon on chromosome 19. A relation with angiogenesis has not been described previously and is new.

### Example 9

### Confirmation of the identity of tag sequences.

A sequence consisting of 15 nucleotides should be enough to identify a particular specific mRNA or gene. To confirm the identity of the tags we developed a RT-PCR using an oligo24dT primer for the RT-reaction. A 6'primer containing the tag sequence itself is used for second strand synthesis. The oligo24dT primer is extended at the 5'site extended with a -21M13 sequence, In the PCR following the RT-reaction -21M13 primer is used for the amplification together with the 5'primer containing the tag sequence. The 5'primer containing the tag sequence is extended with 5 Inosines at its 5'site to enlarge the binding capacity of this primer. The sequence of the amplified fragment can be determined to confirm that this is the gene as identified by the tag sequence. The RT-PCR reactions to confirm the tags were performed on the same tissue samples that were used to prepare the expression profiles of tag sequences.

### Procedure:

Common buffers used throughout the experiments:

10x RT buffer:
- 500 mM TRIS, pH 8.3
- 750 mM KCL
- 30 mM MgCl₂
- 100 mM DTT

10x PCR buffer:
- 200 mM TRIS pH 8.3
- 500 mM KCL
- 1 mg/ml BSA

1 ml TRIzol reagent (Invitrogen Life Technologies, cat. no. 15596) is added to 10-100 mg tissue or approximately 10⁷ cells immediately (tissue is sliced 14µm thick by microtome).
The Total RNA isolation of the samples is performed according to the manufacturers protocol as follows:
- Add 0.2 ml of Chloroform (Merck) and shake the tube vigorously by hand for 15 seconds.
- Incubate for 5 minutes at RT.
- Centrifuge the sample at no more than 12,000 x g for 15 minutes at 4°C.
- Transfer 600µl of the colourless upper aqueous layer to a new tube. The lower organic layer should be red.
- Ad 0.5 ml isopropyl alcohol (Merck) and mix.
- Incubate at room temperature for 10 minutes.
- Centrifuge at no more than 12,000 x g for 15 minutes at 4°C.
- Discard the supernatant and wash the RNA pellet with 1 ml 80% ethanol by vortexing and centrifuge at no more than 7,500 x g at 4°C for 5 minutes and discard the supernatant.
- Place the tube at 56°C for 3 minutes to dry the pellet and proceed with the Poly A⁺ RNA isolation as described in the next section.

The Poly A⁺ mRNA isolation was performed according to the manual of Micro Fasttrack © 2.0 Poly A⁺ mRNA 2.0 isolation kit as provided by the manufacturer (Invitrogen Corporation, Carlsbad USA; cat no K1520),

Subsequently the isolated poly A+ RNA was used as input for analysis in a RT-PCR reaction. The RT-PCR reactions started with the following mixture of ingredients:

| | |
|---|---|
| 21M13POLYT primer (100 ng/µl) | 1.25 µl |
| 10 x RT buffer | 2.0 µl |
| 100 mM dNTP (Pharmacia) | 0.8 µl |
| 20 U RNAsin (Roche) | 0.3 µl |
| dH₂O (Baker) | 0.65 µl |

- Add 10 µl of Poly A⁺ mRNA dilution to 5 µl of RT-mix.
- To anneal the primer to the template incubate the reaction mixture at 65°C for 5 minutes followed by cooling down to room temperature.
- Add 5µl 1U/µl AMV-RT to the reaction mixture and perform the Reverse Transcription by incubating at 42°C for 45 minutes.
- After the Reverse Transcription immediately incubate the mixture at 95°C for 5 minutes to stop the reaction. Then let the reaction cool down to room temperature.
- Add 80 µl of PCR-mix to each reaction mixture (total volume is 100 µl). Prepare the PCR-mix per reaction as follows:
   - 5' primer (100 ng/µl), see table 5 1.0 µl
   - 21M13 (100 ng/µl) 1.0 µl
   - 10x PCR buffer 8,0 µl
   - 100 mM MgCl₂ 2.1 µl
   - Amplitaq 5U/µl (Perkin Elmer) 0.4 µl
   - dH₂O (Baker) 67.5µl
- PCR amplification was performed in a 9700 DNA thermal cycler (Perkin Elmer) according to the following program.
   - 5 minutes 95 °C
   - 1 minute 95 °C; 1 minute 55 °C; 2 minutes 72 °C, for 35 cycles
   - 10 minutes 72 °C

The TA-cloning of the RT-PCR products was performed according to the manual of TOPO TA Cloning® kit as provided by the manufacturer (Invitrogen Corporation, Carlsbad USA; cat no K4600) using the pCR® II-TOPO® Dual promoter vector and the TOP10 One Shot® Cells.
Screening of the clones was performed using PCR with SP6 and T7 primers. As follows:
• Resuspend the colony in 50µl dH₂O (Baker). Add 1 µl of this bacteria suspension to 10 µl of PCR mixture.
• Prepare the PCR-mixture per reaction as follows:

| | |
|---|---|
| SP6 (100 ng/µl) | 0,10 µl |
| T7(100 ng/µl) | 0.10 µl |
| 10x PCR buffer | 1.00 µl |
| 100 mM MgCl₂ | 0.20 µl |
| 100 mM dNTP's (Pharmacia) | 0.08 µl |
| Amplitaq 5U/µl (Perkin Elmer) | 0.04 µl |
| dH₂O (Baker) | 7.48 µl |

• PCR amplification was performed in a 9700 DNA thermal cycler (Perkin Elmer) according to the following program.
- 5 minutes 95 °C
- 30 seconds 95 °C; 30 seconds 55 °C; 1 minutes 72 °C, for 25 cycles
- 10 minutes 72 °C

• Run 5 µl of the Colony-PCR product on a 1.5% agarose 1xTBE gel stained with EthidiumBromide.
• Visualise the amplification products on a UV-illuminator to identify insert-containing clones.

Clones containing insert were sequenced from both directions using SP6 and T7 primers and the ABI Prism Big-Dye terminator cycle sequencing kit (Perkin Elmer Applied Biosystems, Foster City, Calif USA).

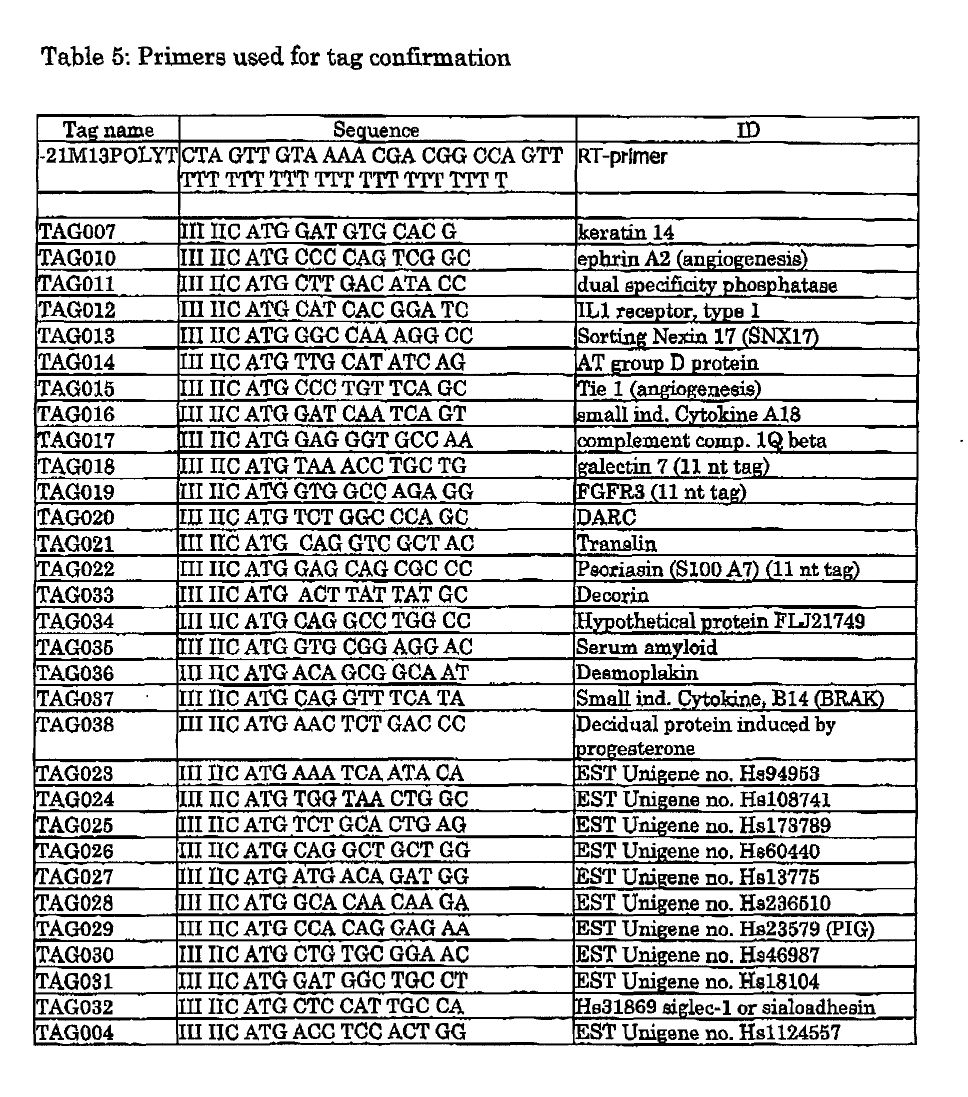

### Results of Confirmation of tag sequences:

Of 18 tag sequences that were analysed with the protocol as described in this example 14 were confirmed with sequences analysis using the RT-PCR with the oligo24dT primer and the tag-based primer as described above (tags designated 004, 007, 011, 012, 014, 015, 016, 017, 022, 025, 029, 030, 032, 036 in table 5). Four tag sequences could not be confirmed based on this method (designated 010, 013, 018, 019). This was probably due to the fact that the tag-based primer was not specific enough or that the polyA tail of the mRNA was not long enough. For one tag an alternative more specific 5'primer was designed to perform a RT-PCR together with -21M13POLYT (tag designated 004). For tag010 a complete specific primer set was designed to perform as well the Reverse Transcription as the amplification. Other tags were confirmed by using a specific RT-PCR primer set followed by a Nested PCR with a specific nested primer set (tags designated 013, 018, 019). Sequence results of all confirmations are listed below and in the figures. The tag sequence in the mRNA sequence if present is shown in bold fonts.

### TAG004 (EST AI217565, genbank number BE466728):

Confirmed with protocol from this example.

CATGACCTCCACTGGAAGAGGGGGCTAGCGTGAGCGCTGATTCTCAACCT
ACCATAACTCTTTCCTGCCTCAGGAACTCCAATAAAACATTTTCCATCCA
(Figure 1)

### TAG007 (keratin 14, genbank number XM_008578):

Confirmed with protocol from this example,

CATGGATGTGCACGATGGCAAGGTGGTGTCCACCCACGAGCAGGTCCTT
CGCACCAAGAACTGAGGCTGCCCAGCCCCGCTCAGGCCTAGGAGGCCCC
CCGTGTGGACACAGATCCCACTGGAAGATCCCCTCTCCTGCCCAAGCACT
TCACAGCTGGACCCTGCTTCACCCTCACCCCCTCCTGGCAATCAATACAG
CTTCATTATCTGAGTTGCTAAAAAAAAAAAAAAAAAAAAAAAA
(Figure 2)

### TAG010 (ephrin A2, genbank number XM_002088):

The RT-PCR with the 5'primer designed on the catg-site (the original primer shown in table 5) gave no confirmation. Most probably the tag-based primer is not specific enough. Ephrin A2 specific primers were used for confirmation.
The tag sequence is not included in the Ephrin specific RT-PCR.

ATCTACCAGCTCATGATGCAGTGCTGGCAGCAGGAGCGTGCCCACCGCCC
CAAGTTCGCTGACATCGTCAGCATCCTGGACAAGCTCATTCGTGCCCCTG
ACTCCCTCAAGACCCTGGCTGACTTTGACCCCCGCGTGTCTATCCGGCTC
CCCAGCACGAGCGGCTCGGAGGGGGTGCCCTTCCGCACGGTGTCCGAGT
GGCTGGAGTCCATCAAGATGCAGCAGTATACGGAGCACTTC
(Figure 3)

### TAG011 (dual specificity phosphatase, genbank number XM_003720):

Confirmed with protocol from this example.

CATGCTTGACATACCTACCAGTATTATTCCCGACGACACATATACATATG
AGAATATACCTTATTTATTTTTGTGTAGGTGTCTGCCTTCACAAATGTCAT
TGTCTACTCCTAGAAGAACCAAATACCTCAATTTTTGTTTTGAGTACTGT
ACTATCCTGTAAATATATCTTAAGCAGGTTTGTTTTCAGCACTGATGGAAA
ATACCAGTGTTGGGTTTTTTTTAGTTGCCAACAGtTGTATGTTTGCTGAT
TATTTATGACCTGAAATAATATATTTCTTCTTCTAAGAAGACATTTTGTTAC
ATAAGGATGACTTTTTTATACAATGGAATAAATTATGGCATTTCTATTG
(Figure 4)

### TAG012 (IL1 receptor, type 1, genbank number XM_002686):

Confirmed with protocol from this example.

CATGCATCACGGATCAATAGACTGTACTTATTTTCCAATAAAATTTTCAA
ACTTTGTACTGTT
(Figure 5)

### TAG013 (ephrin B1, genbank numbers XM_002535, BC002524):

The RT-PCR with the 5'primer designed on the catg-site (the original primer shown in table 5) gave no confirmation. Most probably the tag-based primer is not specific enough. Ephrin B1 specific primers were used for confirmation. The tag sequence is included in the 3'primer of the Ephrin B1 specific RT-PCR fragment. The Nested PCR fragment is shown here and is just located upstream of the tag sequence.

AACTTGCCCTGTGCCTGTGTCCCCCATGCTAGGGGCGGAGGGGTCTTTTC
CTTCTTCTTTCCTACCTACCCCTTTTCTCTTGGCCAGGGGCCTCGTATCCT
ACCTTTCCTTGTCCCCTGGGCTGGCTGCACAGAGGATTGCCCCTTCTCTTT
TCAGAGCTGGCCCTCGATGCCAAATTAGCATTTAGTATTTGCTCAAAGTC
TAAGGACC
(Figure 6)

### TAG014 (AT group D protein, genbank numbers XM_006184, AF230388):

Confirmed with protocol from this example.

CATGTTGCATATCAGGGTGCTCAAGGATTGGAGAGGAGACAAAACCAGG
AGCAGCACAGTGGGGACATCTCCCGTCTCAACAGCCCCAGGCCTATGGGG
GCTCTGGAAGGATGGGCCAGCTTGCAGGGGTTGGGAGGGAGACATCCA
GCTTGGGCTTTCCCCTTTGGAATAAACCATTGGTCTGTCACAAAAAAAAAA
AAAAAAAAAAAAAAA
(Figure 7)

### TAG015 (TIE 1, genbank number XM_002037):

Confirmed with protocol from this example.

CATGCCCTGTTCAGCTACTCCCACTCCCGGCCTGTCATTCAGAAAAAAAT
AAATGTTCTAATAAGCTCCAAAAAAAAAAAAAAAAAAAAAAAAA
(Figure 8)

### TAG016 (small ind. Cytokine A18, genbank numbers XM_008451, Y13710, AF111198):

Confirmed with protocol from this example.

CATGGATCAATCAGTGTGATTAGCTTTCTCAGCAGACATTGTGCCATATG
TATCAAATGACAAATCTTTATTGAATGGTTTTGCTCAGCACCACCTTTTAA
TATATTGGCAGTACTTATTATATAAAGGTAAACCAGCATTCTCAAAAAAA
AAAAAAAAAAA
(Figure 9)

### TAG017 (complement comp. 1Q beta, genbank number XM_010666):

Confirmed with protocol from this example.

CATGGAGGGTGCCAACAGCATCTTTTCCGGGTTCCTGCTCTTTCCAGAT
ATGGAGGCCTGACCTGTGGGCTGCTTCACATCCACCCCGGCTCCCCCTGC
CAGCAACGCTCACTCTACCCCCAACACCACCCCTTGCCCAGCCAATGCAC
ACAGTAGGGCTTGGTGAATGCTGCTGAGTGAATGAGTAAATAAACTCTTC
AAGGCC
(Figure 10)

### TAG018 (galectin 7, genbank numbers NM_002307, U06643):

The RT-PCR with the 5'primer designed on the catg-site (the original primer shown in table 5) gave no confirmation. Most probably the tag-based primer is not specific enough. Galectin 7 specific primers were used for confirmation. The 5'primer used in the Galectin 7 specific RT-PCR contains the tag sequence. The tag sequence is included in the 5'primer of the Galectin 7 specific RT-PCR fragment. The Nested PCR fragment is shown here and is just located downstream of the tag sequence.

CGGCTGGACACGTCGGAGGTGGTCTTCAACAGCAAGGAGCAAGGCTCCT
GGGGCCGCGAGGAGCGCGGGCCGGGCGTTCCTTTCCAGCGCGGGCAGCC
CTTCGAGGTGCTCATCATCGCGTCAGACGACGGCTTCAAGGCCGTGGTTG

GGGACGCCCAGTACCACCACTTCCGCC
(Figure 11)

### TAG019 (FGFR3, genbank numbers NM_022965, NM_000142):

The RT-PCR with the 5'primer designed on the catg-site (the original primer shown in table 5) gave no confirmation. Most probably the tag-based primer is not specific enough. FGFR3 specific primers were used for confirmation. The tag sequence is not included in the Ephrin specific RT-PCR. The Nested PCR fragment is shown here and is located upstream of the tag sequence.

CACAACCTCGACTACTACAAGAAGACAACCAACGGCCGGCTGCCCGTGAA
GTGGATGGCGCCTGAGGCATTATTTGACCGAGTCTACACTCACCAGAGTG
ACGTCTGGTCCTTTGGGGTCCTGCTCTGGGAGATCTTCACGCTGGGGGC
TCCCCGTACCCCGGCATCCCTGTGGAGGAGCTCTTCAAGCTGCTGAAGGA
GGGC
(Figure 12)

### TAG022 (Psoriasin (S100 A7), genbank number XM_048120):

Confirmed with protocol from this example.

CATGGAGCAGCGCCCTGTTCCGGGGGCAGCCAGTGACCCAGCCCCACC
AATGGGCCTCCAGAGACCCCAGGAACAATAAAATGTCTTCTCCCACC
(Figure 13)

### TAG025 (EST Unigene no. Hs173789, genbank numbers XM_018404, AL137262):

Confirmed with protocol from this example.

CATGTCTGCACTGAGAAACTGCATTTCAGTAGCATTTGTCATCCAGCCG
GAAGTTAAAGCACACTTACTTTATTCACCTATTTTTATAATAAACGTTCTT
GCTGCTGTGAAAAAAAAAAAAAAAAAAAAAAAAAAAA
(Figure 14)

### TAG029 (PIG, genbank numbers XM_011453, AJ261830):

Confirmed with protocol from this example.

CATGCCACAGGAGAATTCGGGGATTTGAGTTTCTCTGAATAGCATATAT
ATGATGCATCGGATAGGTCATTATGATTTTTACCATTTCGACTTACATAA
TGAAAACCAATTCATTTTAAATATCAGATTATTATTTTGTAAGTTGTGGAA
AAAGCTAATTGTAGTTTTCATTATGAAGTTTTCCCAATAAACCAGGTATTC
TAAACTTGAAAAAAAAAAAAAAAAAAAAAAAA
(Figure 15)

### TAG030 (EST Unigene no. Hs46987, genbank numbers DG151190, BG057289, BE858276, AV6817591 BE503169):

Confirmed with protocol from this example.

CATGCTGTGCGGAACTGCGTCAGGGCAAATGTCACAGCAGGATTTCCCC
AACCCAGCTCCATCATCACAGACACAGAGGGCTGCAGGGGAGGCCTGCCC
ACTGTTTTGTCGACTCTGCCCTCCTCTGGCAGCATAGATCCTTAGGTGCTC
AATAAAGGTGTGCTGTATTGAAAAAAAAAAAAAAAAAAAAAAAA
(Figure 16)

### TAG032 (SialoAdhesin, also called Siglec 1, genbank number XM_016245):

Confirmed with protocol from this example.

CATGCTCCATTGCCAGACTCTTGCTGGGAGCCCGTCCAGAATGTCCTCC
CAATAAAACTCCATCCTATGACGCAAAAAAAAAAAAAAAAAAAAAAA
(Figure 17)

### TAG036 (Desmoplakin, genbank numbers XM_004463, NM_004415, AF139065):

Confirmed with protocol from this example.

CATGACAGCGGCAATCTTTTCTTTGGTCAAAGTTTTCTGTTATTTTGCT
TGTCATATTCGATGTACTTTAAGGTGTCTTTATGAAGTTTGCTATTCTGGC
AATAAACTTTTAGACTTTAAAAAAAAAAAAAAAAAAAAAAAA

(Figure 18)

### Example 10

### Determination of the gene expression levels of the tag sequences in skin samples

To get a feeling for the use of the tag sequences as markers for angiogenesis process skin samples with (5 different samples) and without (2 control samples) Kaposi's Sarcoma lesions were analysed for the expression level of the genes identified by the tag sequences.

### Procedure:

1 ml TRIzol reagent (Invitrogen Life Technologies, cat. no. 15596) is added to 10-100 mg tissue or approximately 10⁷ cells immediately (tissue is sliced 14µm thick by microtome).
The Total RNA isolation of the samples is performed according to the manufacturers protocol as follows:
- Add 0.2 ml of Chloroform (Merck) and shake the tube vigorously by hand for 15 seconds.
- Incubate for 5 minutes at RT.
- Centrifuge the sample at no more than 12,000 x g for 16 minutes at 4°C.
- Transfer 600µl of the colourless upper aqueous layer to a new tube. The lower organic layer should be red.
- Ad 0.5 ml isopropyl alcohol (Merck) and mix.
- Incubate at room temperature for 10 minutes.
- Centrifuge at no more than 12,000 x g for 15 minutes at 4°C.
- Discard the supernatant and wash the RNA pellet with 1 ml 80% ethanol by vortexing and centrifuge at no more than 7,500 x g at 4°C for 5 minutes and discard the supernatant.
- Place the tube at 56°C for 3 minutes to dry the pellet and proceed with the DNase treatment as described in the next section.

To make sure no genomic DNA exists in the Total RNA isolate we perform a DNase treatment. Protection of RNA against RNase activity of DNase I is done by the addition of RNasin to the DNase reaction. The DNase treatment was performed as follows:
- After TRizol Total RNA isolation resuspend the pellet in 88 µl dH₂O.
- Add sequentially to the RNA solution:
   - 10 µl 10x DNase Buffer (Ambion)
   - 1 µl 40U/µl RNasin (Roche)
   - 1 µl 10 U/µl DNase I (Roche)
- Incubate at 37°C for 1 hr.
- Raise sample volume to 200µl by adding 100 µl dH₂O.
- Add 200 µl cold Phenol/Chloroform pH8 (PC8) and mix thoroughly.
- Centrifuge full speed for 5 minutes at room temperature in a microcentrifuge.
- Transfer the aqueous top layer to a new microcentrifuge tube add sequentially:
   - 3 µl glycogen (Roche)
   - 100 µl 10 M ammonium acetate
   - 700 µl 100% ethanol
- Mix thoroughly and centrifuge at 25,000 x g for 15 minutes at 4°C and decant the supernatant.
- Wash twice by adding 700 µl 80 % ethanol. Mix thoroughly and centrifuge at 25,000 x g for 5 minutes at 4°C and decant the supernatant.
- Dry the pellet for 5 minutes at 56°C and resuspend in 11 µl dH₂O.
- Dilute 1 µl of the RNA isolate in 140 µl dH₂O and calculate the RNA concentration and yield of the isolate through OD₂₆₀ measurement. The yield of DNase treated Total RNA should at least be 13.5µg for the determination of the complete TAG expression profile.
- Prepare a solution of the RNA isolate with a concentration of 50 ng/µl. This is the starting RNA solution for the series of dilution for the TAG specific RT-PCR.

Per sample of DNase treated Total RNA 18 TAG specific RT-PCR/Nested PCR reactions are performed in series of dilution of the RNA. For each sample five control RT-PCR's will be performed in series of dilution namely HIV-1 GAG (SK39/145), GAPDH +RT (in duplo) and no-RT reaction (in duplo). The following dilution scheme was used to derived the samples in serial dilution that are analysed with the RT-PCR:
1. 270 µl 50 ng/µl DNase treated Total RNA DNase treated.
   10 µl 50 ng/µl solution as input in RT-PCR of each primerset = 500ng.
2. 27 µl 50ng/µl Total RNA 10x diluted = 270 µl 5 ng/µl.
   10 µl 5 ng/µl solution as input in RT-PCR of each primerset = 50 ng.
3. 27 µl 5 ng/µl Total RNA 10x diluted = 270 µl 0.5 ng/µl.
   10 µl 0.5 ng/µl solution as input in RT-PCR of each primerset = 5 ng.
4. 27 µl 0.5 ng/µl Total RNA 10x diluted = 270 µl 0.05 ng/µl.
   10 µl 0.05 ng/µl solution as input in RT-PCR of each primerset = 0.5 ng.
5. 27 µl 0.05 ng/µl Total RNA 10x diluted = 270 µl 0.005 ng/µl.
   10 µl 0.005 ng/µl solution as input in RT-PCR of each primerset = 0.05 ng.
6. 27 µl 0.005 ng/µl Total RNA 10x diluted = 270 µl 0.0005 ng/µl.
   10 µl 0.0005 ng/µl solution as input in RT-PCR of each primerset = 0.005 ng.
7. 27µl 0.0005 ng/µl Total RNA 10x diluted = 270 µl 0.00005 ng/µl.
   10 µl 0.00005 ng/µl solution as input in RT-PCR of each primerset = 0.0005 ng.
8. 27 µl 0.00005 ng/µl Total RNA 10x diluted = 270 µl 0.000005 ng/µl.
   10 µl 0.000005 ng/µl solution as input in RT-PCR of each primerset = 0.00005 ng.

TAG specific RT-PCR on Total RNA series of dilution using AMV-RT was performed as follows. The reverse Transcription Reactions of all the TAGs on DNase treated Total RNA in series of dilution are performed in 96 wells PCR plates. Ten µl of each Total RNA dilution is used as input for the RT PCR. The reaction volume of the Reverse Transcription is 20 µl and contains dNTP's, MgCl2 and RNasin,
• Prepare the RT-mix per reaction as follows:

| | |
|---|---|
| 3' primer (100 ng/yl) see table 6 | 1.25 µl |
| 10 xRT buffer | 2.0 µl |
| 100 mM dNTP (Pharmacia) | 0,8 µl |
| 20 U RNAsin (Roche) | 0.3 µl |
| dH₂O (Baker) | 0.65 µl |

• Add 10 µl of Total RNA dilution to 5 µl of RT-mix.
• To anneal the primer to the template incubate the reaction mixture at 65°C for 5 minutes followed by cooling down to room temperature.
• Add 5µl 1U/µl AMV-RT to the reaction mixture and perform the Reverse Transcription by incubating at 42°C for 45 minutes.
• After the Reverse Transcription immediately incubate mixture at 95°C for 5 minutes to stop the reaction. Then let the reaction cool down to room temperature.
• Add 80 µl of PCR-mix to each reaction mixture (total volume is 100 µl). Prepare the PCR-mix per reaction as follows;

| | |
|---|---|
| 5' primer (100 ng/µl) see table 6 | 1.0 µl |
| 10x PCR buffer | 8.0 µl |
| 100 mM MgCl₂ | 1.9 µl |
| Amplitaq 5U/µl | 0.4 µl |
| dH₂O (Baker) | 68.7µl |

• PCR amplification is performed in a 9700 DNA thermal cycler (Perkin Elmer) according to the following program.
- 5 minutes 95 °C
- 1 minute 95 °C; 1 minute 55 °C; 2 minutes 72 °C, for 35 cycles
- 10 minutes 72 °C

Subsequent to this first round of amplification a second round, nested, amplification was performed. TAG specific second round nested PCR on RT-PCR product was performed as follows:

Add 5 µl of the TAG RT-PCR product in 45 µl of the Nested-PCR mix.
• Prepare the Nested-PCR mix per reaction as follows:

| | |
|---|---|
| 5' nested primer (100 ng/µl), see table 7 | 0.5 µl |
| 3' nested primer (100 ng/µl), see table 7 | 0.6 µl |
| 10x PCR, buffer | 5.0 µl |
| 100 mM MgCl₂ | 1.25 µl |
| 100 mM dNTP (Pharmacia) | 0.4 µl |
| Amplitaq 5U/µl | 0.2 µl |
| dH₂O (Baker) | 37.15µl |

The combination of primers used for amplification of the genes identified by the tags and the length of the amplified fragment is given in table 8.
• PCR amplification is performed in a 9700 DNA thermal cycler (Perkin Elmer) according to the following program.
- 5 minutes 95 °C
- 1 minute 95 °C; 1 minute 55 °C; 2 minutes 72 °C, for 25 cycles
- 10 minutes 72 °C

• Run 10 µl of the Nested-PCR product on a 1.5% agarose 1xTBE gel stained with EthidiumBromide.
• Visualization of the TAG amplified fragments in the dilution series on a UV-illuminator reveals the level of expression by determining the highest dilution still giving a positive signal.

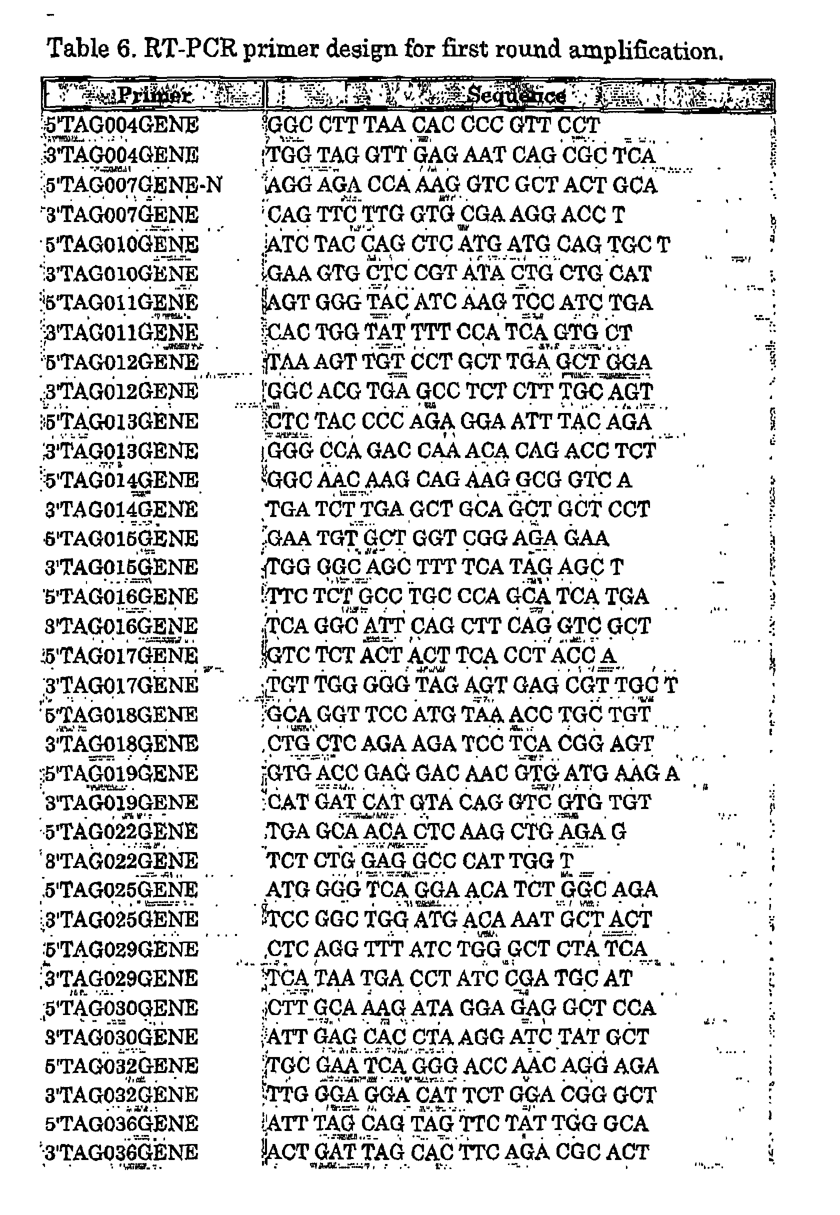

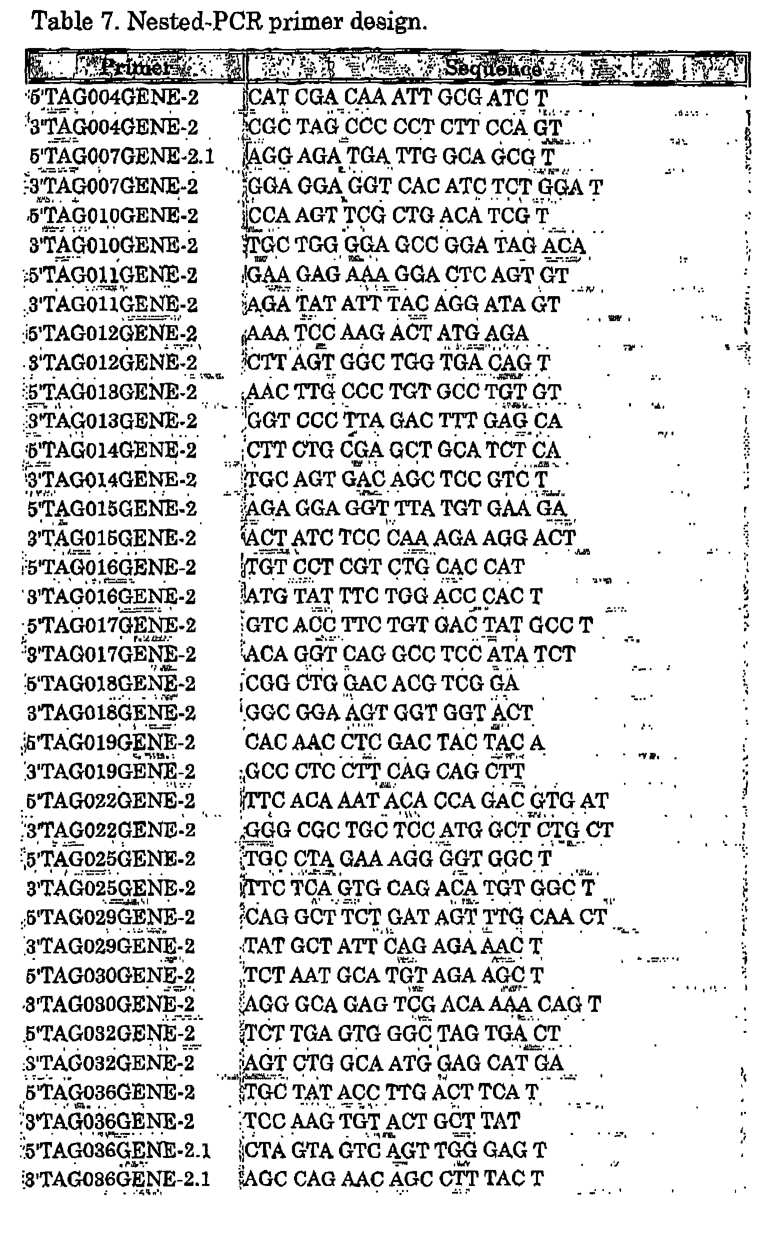

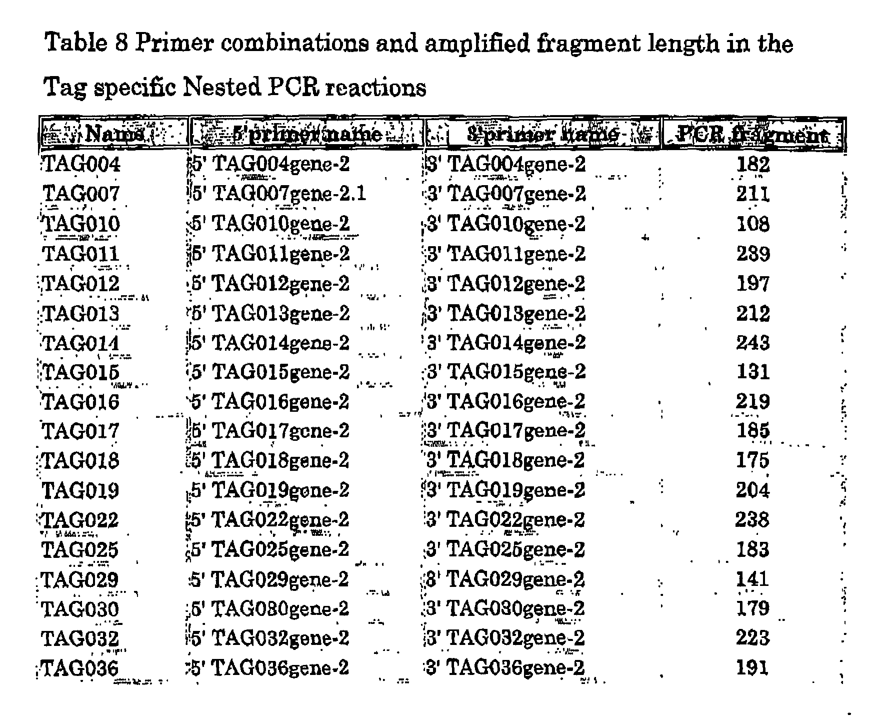

### Results

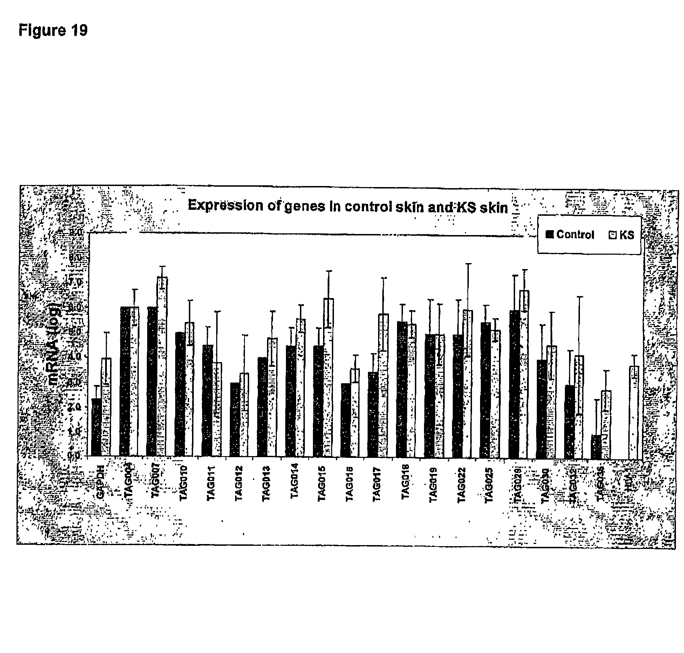

The results of determination of the expression levels of the genes identified by the tag sequences are depicted in figure 19. The data clearly indicate that a number of the genes identified by the tag sequences have a higher expression in the skin samples with Kaposi's Sarcoma lesions compared to normal skin: tag007, tag010, tag012, tag013, tag014,, tag015, tag016, tag017, tag022, tag029, tag030, tag032 and tag036.

### Example 11

### Determination of the gene expression levels of the tag sequences in peripheral blood mononuclear cell (PBMC) samples

To get a feeling for the use of the tag sequences as markers for angiogenesis process in samples not from the location of the angiogenesis process, i.e. the Kaposi's Sarcoma in the skin or another tumour in the body but at an accessible sample from the blood (FBMC's) the expression of 5 tag identified genes was determined in PBMC samples. The PBMC samples were from the blood of patients with (4 different samples) and without (2 control samples) Kaposi's Sarcoma lesions and were analysed for the expression level of 5 tag identified genes in PBMC's.

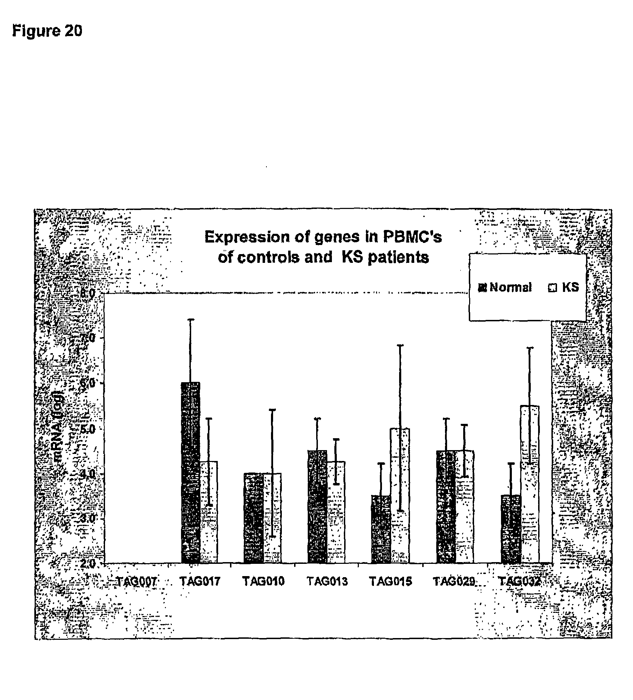

The procedure of the example was identical as described in example 10, with the exception that PBMC samples were used (approximately 10 million cells per sample, ranging from 2.5 to 50 million) instead of skin biopsies. The genes that were analysed are identified by tag007, tag017, tag010, tag013, tag015, tag029 and tag032. The results of the analysis are depicted in figure 20. It is clear from these data that the elevated expression in the tumour sites of the genes identified by tag015 (TIE 1) and tag032 (Salioadhesin or Siglec 1) is paralleled in the blood cell fraction, i.e. PBMC.

### Results

The data clearly show that the over expression of the gene identified by tag007 (Keratin 14) in skin samples (see example 5) is not paralleled in blood. In contrast, in the samples tested in this example the gene identified by tag007 is not expressed at all in the blood compartment. This shows that no tumour cells expressing tag007 are present in blood. As a consequence, measurement of tag007 in the blood could be a good indicator for the presence of these tumour cells in the blood, and thus a marker for circulating cancer cells that can cause metastasis.
The up regulation of genes identified by tag015 (TIE 1) and tag032 (Saliodhesin or Siglec 1) in skin samples is clearly paralleled in the blood. These two tags are higher expressed in blood from patients with tumours compared to healthy individuals. This up-regulation is due to up-regulation of expression in typical blood cells. The up-regulation cannot be due to the presence of tumour cells that express tag015 and tag032 in the blood, because the absence of expression of the gene identified by tag007 in the blood shows that no tumour cells are present in blood, as explained above. This means that measurement of expression of tag015 and/or tag032 in the blood indicates the presence of a tumour somewhere else in the body. Furthermore, measurement of expression of genes identified by tag015 and/or tag032 during anti-tumour therapy and/or anti-angiogenesis therapy can be used to monitor the efficacy of this treatment.

### Conclusions

The paralleled up-regulation of the genes identified with tag015 (TIE 1) and tag032 (Salioadhesin or Siglec 1) in both the tumour and the blood, enables the monitoring of the efficacy of a therapy aimed at decreasing the growth of a tumour, in particular anti-angiogenic tumour treatment. This follows the reasoning that if these two genes are markers in PBMC for blood vessel formation in a tumour in another site in the body, these two markers in blood will also decrease with the decrease of this blood vessel growth at the tumour site.
The genes identified by tag007 (Keratin 14), tag015 (TIE 1) and tag032 (Salioadhesin or Siglec 1) have different expression in the blood of patients with a tumour compared to normal individuals. Therefore these genes, in particular the expression thereof, can be used to screen a population at risk for the presence of tumour in individual members of that population.

All the genes identified by tags in this study that have changed expression levels comparing normal to tumour tissue, i.e. tag007, tag010, tag012, tag013, tag014, tag015, tag016, tag017, tag022, tag029, tag030, tag032 and tag036 are encoding potential target molecules for therapeutic compound with anti-angiogenic effects applicable in tumour treatment, and/or these genes encode potential target molecules that can be potential target molecules for therapeutic compounds that stimulate the growth of blood vessel, for instance in the treatment of heart and coronary disease.

### Brief description of the drawings

Figure 1: Sequence involved in angiogenesis. A change of expression of this sequence after a certain treatment indicates that said treatment is effective. This sequence is identical to an EST sequence identified from human foetal heart (GenBank acc. # AI217565 and others), which in turn matches a predicted exon on chromosome 19. A relation with angiogenesis has not been described previously.

Figure 2-18: Sequences which are identified by name and Genbank numbers (NCBI database), Other identification can be found in tables 1-4 (Unigene numbers) that can be found in the SAGE databases of NCBI.

Figure 19. Mean expression levels and standard deviation depicted as log dilution factor that still is positive starting with 500 ng of total RNA isolated from 5 skin samples with Kaposi's Sarcoma (light bare) and 2 control, normal skin samples (dark bars).

Figure 20. Mean expression levels and standard deviation depicted as log dilution factor that still is positive starting with 500 ng of total RNA isolated from 4 PBMC samples of patients with Kaposi's Sarcoma (light bars) and 2 control, normal PBMC samples (dark bars).

## Claims

1. A method for determining whether a treatment is effective in changing a status of a certain set of target cells in an individual comprising:
- obtaining a sample from said individual after initiation of said treatment, and
- determining whether said sample comprises an expression product of at least one marker gene.

2. A method according to claim 1, wherein said target cells comprise a tumor cell.

3. A method according to claim 1 or 2, wherein said sample comprises at least one of said target cells.

4. A method according to any one of claims 1-3, wherein said sample is obtained within a week of initiation of said treatment.

5. A method according to any one of claims 1-4, wherein said sample is obtained within two days of initiation of said treatment.

6. A method according to any one of claims 1-5, wherein said marker gene comprises a gene involved in the generation, maintenance and/or breakdown of blood vessels.

7. A method according to any one of claims 1-6, wherein said marker gene comprises a sequence as depicted in table 1 or 2.

8. A method according to any one of claims 1-7, wherein said marker gene comprises a sequence as depicted in figure 1-18, or a part or analogue thereof.

9. A method according to any one of claims 1-8, wherein expression of said marker gene is quantified.

10. A method according to any one of claims 1-9, further comprising comparing expression of said marker gene with a reference.

11. A method according to any one of claims 2-10, wherein said tumor comprises Kaposi's Sarcoma.

12. Use of a nucleic acid comprising a sequence as depicted in figure 1-18 and/or table 1 or 2, or a part or analogue thereof, in an expression product detection method.

13. Use of a proteinaceous molecule capable of specifically binding a protein encoded by a nucleic acid comprising a sequence as depicted in figure 1-18 and/or table 1 or 2, or a part or analogue thereof, in a detection method.

14. A use according to claim 12 or claim 13 for determining the presence of a tumor cell in an individual.

15. A use according to claim 12 or claim 13 for determining the presence of a site of angiogenesis in an individual.

16. A use according to claim 12 or claim 13, for determining whether a treatment is effective in changing the status of a certain set of target cells in an individual.

17. A use according to any one of claims 12-16, for determining whether a treatment is effective in counteracting a tumor in an individual.

18. A use according to claim 14 or 17, wherein said tumor comprises Kaposi's Sarcoma.

19. A method for determining whether an individual comprises a tumor cell and/or a site of angiogenesis comprising:
- obtaining a sample from said individual, and
- determining whether said sample comprises an expression product of at least one marker gene.

20. A method according to claim 19, wherein said marker gene comprises a sequence as depicted in table 1 or 2 and/or figure 1-18, or a part or analogue thereof.

21. A method for determining whether an individual comprises a non-hemopoietic tumor cell and/or a site of angiogenesis, said method comprising determining whether a hemopoietic cell from said patient comprises an altered amount of an expression product of a marker gene as compared with a reference value.

22. A method according to claim 21, wherein said marker gene comprises a gene involved in angiogenesis,

23. A method according to claim 21 or 22, wherein said gene comprises a sequence as depicted in table 1 or 2, and/or figures 1-18, or a part or analogue thereof.

24. A method according to any one of claims 21-23, wherein said hemopoietic cell comprises a peripheral blood mononuclear cell.

25. A method for determining whether a treatment is effective in altering an angiogenic process in an individual comprising:
- obtaining a sample from said individual after initiation of said treatment, and
- determining whether said sample comprises an expression product of at least one marker gene.

26. A method according to claim 25, wherein said treatment comprises counteracting angiogenesis in said individual.

27. A method according to claim 25 or claim 26, wherein said marker gene comprises a sequence as depicted in table 1, or table 2, or figures 1-18, or a part or analogue thereof.

28. A method according to any one of claims 25-27, wherein said treatment involves the use of at least one of the following drugs: 2ME2, Angiostatin, Angiozyme, Anti-VEGF RhuMAb, Apra (CT-2584), Avicine, Benefin, BMS275291, Carboxyamidotriazole, CC4047, CC5013, CC7085, CDC801, CGP-41251 (PKC 412), CM101, Combretastatin A-4 Prodrug, EMD 121974, Endostatin, Flavopiridol, Genistein (GCP), Green Tea Extract, IM-862, ImmTher, Interferon alpha, Interleukin-12, Iressa (ZD1839), Marimastat, Metastat (Col-3), Neovastat, Octreotide, Paclitaxel, Penicillamine, Photofrin, Photopoint, PI-88, Prinomastat (AG-3340), PTK787 (ZK22684), RO317453, Solimastat, Squalamine, SU 101, SU 5416, SU-6668, Suradista (FCE 26644), Suramin (Metaret), Tetrathiomolybdate, Thalidomide, TNP-470, Vitaxin.

29. A method according to any one of claims 1-11, 19-20, or 23-26 wherein said sample is a blood sample.

30. A method according to any one of claims 1-11, 19-20, or 24-27, wherein said sample comprises a peripheral blood mononuclear cell.

31. A method according to any one of claims 21-30, wherein said expression product comprises a TIE 1 sequence, a Salioadhesin or Siglec 1 sequence, a sequence as depicted in figure 8 or 17, or a part or analogue thereof.

32. Use of a PBMC expressed Keratin 14 sequence, TIE 1 sequence, Salioadhesin or Siglec 1 sequence, a sequence as depicted in figure 2, 8, or 17, or a part or analogue thereof, as an indicator for angiogenesis.

33. Use of a PBMC expressed Keratin 14 sequence, TIE 1 sequence, Salioadhesin or Siglec 1 sequence, a sequence as depicted in figure 2, 8, or 17, or an analogue thereof, for determining the presence of a tumor cell in an individual.

34. An isolated Keratin 14 sequence, TIE 1 sequence, Salioadhesin or Siglec 1 sequence, a sequence as depicted in figure 2, 8, or 17, or an analogue thereof, for use in a diagnostic method.

35. A diagnostic kit comprising a nucleic acid comprising a sequence as depicted in figure 1-18 and/or table 1 or 2, or a part or analogue thereof, and/or a proteinaceous molecule capable of specifically binding a protein encoded by said nucleic acid or said part or analogue.

36. A diagnostic kit according to claim 35 comprising a Keratin 14 sequence, and/or a TIE 1 sequence, and/or a Salioadhesin or Siglec 1 sequence, and/or a sequence as depicted in figure 2, 8 or 17, or a part or analogue thereof.

37. Use of a diagnostic kit according to claim 35 or 36 for determining whether a treatment is effective in changing the status of a certain set of target cells in an individual and/or altering an angiogenic process in an individual.

38. Use of a diagnostic kit according to claim 35 or 36 for determining whether an individual comprises a tumor cell and/or a site of angiogenesis.

39. Use of an expression product of a gene comprising a sequence as depicted in figure 1-18, table 1 or table 2 as a drugtarget.

40. A compound capable of altering the activity of Salioadhesin or Siglec 1, TIE 1, Keratin 14, and/or the expression of Salioadhesin or Siglec 1, TIE 1, and/or Keratin 14 in a cell.

41. Use of a compound according to claim 40 for the preparation of a medicament.
